# EUROPEAN PATENT SPECIFICATION

(11) **EP 1 543 003 B1**
(45) Date of publication and mention of the grant of the patent: **17.05.2006**
(21) Application number: 03766355.6
(22) Date of filing: 29.07.2003
(51) Int. Cl.: C07D 471/04, A61K 31/437, A61P 13/12, A61P 1/16, C07D 235/00, C07D 221/00

(54) **IMIDAZO¬1,2-A|PYRIDINES**
IMIDAZO¬1,2-A|PYRIDINE
IMIDAZO¬1,2-A|PYRIDINES

(30) Priority: 31.07.2002 GB 0217783
(43) Date of publication of application: 22.06.2005
(73) Proprietor: SMITHKLINE BEECHAM CORPORATION, Philadelphia, Pennsylvania 19101 (US)
(72) Inventor: DODIC, Nerina Laboratoire GlaxoSmithKline, F-91940 Les Ulis (FR); GELLIBERT, Francoise J. Lab. GlaxoSmithkline, 25 avenue de Quebec F-91940 Les Ulis (FR)
(74) Representative: Rowden, Janette Yvonne
(86) International application number: PCT/EP2003/008390
(87) International publication number: WO 2004/013138

(56) References cited:
- WO-A-02/055077
- DATABASE CA [Online] CHEMICAL ABSTRACTS SERVICE, COLUMBUS, OHIO, US; MIYATA, KAZUYOSHI ET AL: "Phosphate derivatives for treatment of nephritis" retrieved from STN Database accession no. 131:317778 XP002262382 & JP 11 302177 A (OHTSUKA PHARMACEUTICAL CO., LTD., JAPAN) 2 November 1999 (1999-11-02)

## Description

This invention relates to novel imidazopyridine derivatives which are inhibitors of the transforming growth factor, ("TGF")-β signalling pathway, in particular, the phosphorylation of smad2 or smad3 by the TGF-β type I or activin-like kinase ("ALK")-5 receptor, methods for their preparation and their use in medicine, specifically in the treatment and prevention of a disease state mediated by this pathway.

TGF-β1 is the prototypic member of a family of cytokines including the TGF-βs, activins, inhibins, bone morphogenetic proteins and Müllerian-inhibiting substance, that signal through a family of single transmembrane serine/threonine kinase receptors. These receptors can be divided into two classes, the type I or activin like kinase (ALK) receptors and type II receptors. The ALK receptors are distinguished from the type II receptors in that the ALK receptors (a) lack the serine/threonine rich intracellular tail, (b) possess serine/threonine kinase domains that are very homologous between type I receptors, and (c) share a common sequence motif called the GS domain, consisting of a region rich in glycine and serine residues. The GS domain is at the amino terminal end of the intracellular kinase domain and is critical for activation by the type II receptor. Several studies have shown that TGF-β signaling requires both the ALK and type II receptors. Specifically, the type II receptor phosphorylates the GS domain of the type I receptor for TGF-β, ALK5, in the presence of TGF-β. The ALK5. in turn, phosphorylates the cytoplasmic proteins smad2 and smad3 at two carboxy terminal serines. The phosphorylated smad proteins translocate into the nucleus and activate genes that contribute to the production of extracellular matrix. Therefore, preferred compounds of this invention are selective in that they inhibit the type I receptor and thus matrix production.

Surprisingly, it has now been discovered that a class of novel imidazopyridine derivatives function as potent and selective non-peptide inhibitors of ALK5 kinase.

According to a first aspect, the invention provides a compound of formula (I), a pharmaceutically acceptable salt, solvate or derivative thereof: 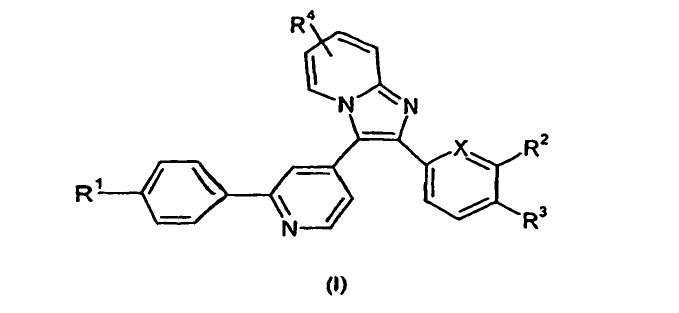 wherein
X is N or CH;
R¹ is selected from hydrogen, C₁₋₆alkyl, C₁₋₆alkenyl, C₁₋₆alkoxy, halo, cyano, perftuoro C₁₋₆alkyl, perfluoroC₁₋₆alkoxy, -NR⁵R⁶, -(CH₂)ₙNR⁵R⁶, -O(CH₂)ₙOR⁷, -O(CH₂)ₙ-Het, -O(CH₂)ₙNR⁵R⁶, -CONR⁵R⁶, -C(O)R⁷. -CO(CH₂)ₙNR⁵R⁶, -SO₂R⁷, -SO₂NR⁵R⁶, -NR⁵SO₂R⁷, -NR⁵COR⁷ and -O(CH₂)ₙCONR⁵R⁶;
R² is hydrogen, C₁₋₆alkyl, halo, cyano or perfluoroC₁₋₆alkyl;
R³ is hydrogen or halo;
R⁴ is hydrogen, halo, C₁₋₆alkyl or -NR⁵R⁶;
R⁵ and R⁶ are independently selected from hydrogen, C₁₋₆alkyl, perfluoroC₁₋₆alkyl, Het or C₁₋₄alkoxyC₁₋₄alkyl; or R⁵ and R⁶ together with the nitrogen atom to which they are attached form a 3, 4, 5, 6 or 7-membered saturated or unsaturated ring which may contain one or more heteroatoms selected from N, S or O, and wherein the ring may be further substituted by one or more substituents selected from halo (such as fluoro, chloro, bromo), cyano, -CF₃, hydroxy, -OCF₃. C₁₋₆alkyl and C₁₋₆alkoxy;
R⁷ is hydrogen or C₁₋₆alkyl;
Het is a 5 or 6-membered C-finked heterocyctyl group which may be saturated, unsaturated or aromatic, which may contain one or more heteroatoms selected from N, S or O and which may be substituted by C₁₋₆alkyl; and
n is 1-4.

The term "C₁₋₆alkyl" as used herein, whether on its own or as part of a group, refers to a straight or branched chain saturated aliphatic hydrocarbon radical of 1 to 6 carbon atoms, unless the chain length is limited thereto, including, but not limited to methyl, ethyl, n-propyl, isopropyl, n-butyl, see-butyl, isobutyl, tert-butyl, pentyl and hexyl.

The term "alkenyl" as a group or part of a group refers to a straight or branched chain mono- or poly-unsaturated aliphatic hydrocarbon radical containing the specified number(s) of carbon atoms. References to "alkenyl" groups include groups which may be in the E- or Z-form or mixtures thereof.

The term "alkoxy" as a group or part of a group refers to an alkyl ether radical, wherein the term "alkyl" is defined above. Such alkoxy groups in particular include methoxy, ethoxy, n-propoxy, *iso*-propoxy, n-butoxy, *iso*-butoxy, *sec*-butoxy and *tert-*butoxy.

The term "perfluoroalkyl" as used herein includes compounds such as trifluoromethyl.

The term "perfluoroalkoxy" as used herein includes compounds such as trifluorornethoxy.

The terrns "halo" or "halogen" are used interchangeably herein to mean radicals derived from the elements chlorine, fluorine, iodine and bromine.

The term "heterocyclyl" as used herein includes cyclic groups containing 5 to 7 ring-atoms up to 4 of which may be hetero-atoms such as nitrogen, oxygen and sulfur, and may be saturated, unsaturated or aromatic. Examples of heterocyclyl groups are furyl, thienyl, pyrrolyl, pyrrolinyl, pyrrolidinyl.. imidazolyl, dioxolanyl, oxazolyl, thiazolyl, imidazolyl, imidazolinyl, imidazolidinyl, pyrazolyl, pyrazolinyl, pyrazolidinyl, isoxazolyl, isothiazolyl, oxadiazolyl, triazolyl, thiadiazolyl, pyranyl, pyridyl, piperidinyl, dioxanyl, morpholino, dithianyl, thiomorpholino, pyridazinyl, pyrimidinyl, pyrazinyl, piperazinyl, sulfolanyl, tetrazolyl, triazinyl, azepinyl, oxazepinyl, thiazepinyl, diazepinyl and thiazolinyl. In addition, the term heterocyclyl includes fused heterocyclyl groups, for example benzimidazolyl, benzoxazolyl, imidazopyridinyl, benzoxazinyl, benzothiazinyl, oxazolopyridinyl, benzofuranyl, quinolinyl, quinazolinyl, quinoxalinyl, dihydroquinazolinyl, benzothiazolyl, phthalimido, benzofuranyl, benzodiazepinyl, indolyl and isoindolyl.

Preferably X is N.

Preferably R¹ is -NR⁵R⁶, -(CH₂)ₙNR⁵R⁶, -O(CH₂)ₙNR⁵R⁶, -O(CH₂)ₙ-Het (preferably imidazolyl), -CONR⁵R⁶, -CO(CH₂)ₙNR⁵R⁶ or -SO₂R⁷. More preferably R¹ is -NR⁵R⁶, -O(CH₂)ₙ-Het (preferably imidazolyl) or -CONR⁵R⁶.

Preferably R² is hydrogen, C₁₋₆alkyl, chloro or fluoro. More preferably R² is hydrogen, methyl, chloro or fluoro. More preferably R² is methyl.

Preferably R³ is hydrogen or fluoro.

Preferably, when X is N, R² is methyl. More preferably when X is N and R² is methyl, R³ is H.

Preferably R⁴ is hydrogen, C₁₋₆alkyl or halo. More preferably, R⁴ is hydrogen, methyl or chloro.

Preferably, R⁵ and R⁶ are independently hydrogen, methyl or Het; or R⁵ and R⁶ together with the nitrogen atom to which they are attached form a 3, 4, 5, 6 or 7 membered saturated or unsaturated ring which may contain one or more heteroatoms selected from N, S or O, and wherein the ring may be further substituted by one or more substitutents selected from halo (such as fluoro, chloro, bromo), cyano, -CF₃, hydroxy, -OCF₃, C₁₋₄alkyl and C₁₋₄alkoxy.

More preferably, R⁵ and R⁶ are independently hydrogen, methyl or tetrahydropyranyl; or R⁵ and R⁶ together with the nitrogen atom to which they are attached form a morpholine, pyrrolidine, piperazine ring, each of which may be substituted by halo (such as fluoro, chloro, bromo), cyano, -CF₃, hydroxy, -OCF₃, C₁₋₄alkyl or C₁₋₄alkoxy.

It will be appreciated that the present invention is intended to include compounds having any combination of the preferred groups listed hereinbefore.

Preferably
X is N;
R¹ is -NR⁵R⁶, -(CH₂)ₙNR⁵R⁶, -O(CH₂)ₙNR⁵R⁶, -O(CH₂)ₙ-Het (preferably imidazolyl), -CONR⁵R⁶, -CO(CH₂)ₙNR⁵R⁶ or -SO₂R⁷;
R² is hydrogen, C₁₋₆alkyl, chloro or fluoro;
R³ is hydrogen or fluoro;
R⁴ is hydrogen, C₁₋₆alkyl or halo;
R⁵ and R⁶ are independently hydrogen, methyl or Het; or R⁵ and R⁶ together with the nitrogen atom to which they are attached form a 3, 4, 5, 6 or 7 membered saturated or unsaturated ring which may contain one or more heteroatoms selected from N, S or O, and wherein the ring may be further substituted by one or more substitutents selected from halo (such as fluoro, chloro, bromo), cyano, -CF₃, hydroxy, -OCF₃, C₁₋₄alkyl and C₁₋₄alkoxy;
R⁷ is hydrogen or C₁₋₆alkyl;
Het is a 5 or 6-membered C-linked heterocyclyl group which may be saturated, unsaturated or aromatic, which may contain one or more heteroatoms selected from N, S or O and which may be substituted by C₁₋₆alkyl; and
n is 1-4.

Compounds of formula (I) which are of special interest as agents useful in the treatment or prophylaxis of disorders characterised by the overexpression of TGF-β are:
3-[2-(4-methanesulfonyl-phenyl)-pyridin-4-yl]-2-(6-methyl-pyridin-2-yl)- imidazo[1,2-a]pyridine (Example 6);
3-[2-(4-(morpholin-4-yl)-phenyl)-pyridin-4-yl]-2-pyridin-2-yl-imidazo[1,2-a]pyridine (Example 14);
3-{2-[4-(4-methylpiperazin-1-yl)-phenyl]-pyridin-4-yl}-2-pyridin-2-yl-imidazo[1,2-a]pyridine (Example 15);
2-(6-methyl-pyridin-2-yl)-3-[2-(4-(morpholin-4-ylmethyl)phenyl)-pyridin-4-yl]-imidazo[1,2-a]pyridine (Example 16);
2-(6-methyl-pyridin-2-yl)-3-{2-[4-((morpholin-4-yl)carbonyl)phenyl]-pyridin-4-yl}-imidazo[1,2-a]pyridine (Example 29);
2-(pyridin-2-yl)-3-{2-[4-(2-(pyrrolidin-1-yl)ethoxy)phenyl]-pyridin-4-yl}-imidazo[1,2-a]pyridine (Example 34);
7-methyl-2-(6-methyl-pyridin-2-yl)-3-{2-[4-(2-(pyrrolidin-1-yl)ethoxy)phenyl]-pyridin-4-yl}-imidazo[1,2-a]pyridine (Example 38);
7-methyl-2-(6-methyl-pyridin-2-yl)-3-{2-[4-((1-methyl-imidazol-4-yl)methyloxy)phenyl]-pyridin-4-y}-imidazo[1,2-a]pyridine (Example 41); and
7-methyl-2-(6-methyl-pyridin-2-yl)-3-{2-[4(aminocarbonylmethyloxy)phenyl]-pyridin-4-yl}-imidazo[1,2-*a*]pyridine (Example 43);
and pharmaceutically acceptable salts, solvates and derivatives thereof.

For the avoidance of doubt, unless otherwise indicated, the term substituted means substituted by one or more defined groups. In the case where groups may be selected from a number of alternative groups, the selected groups may be the same or different.

For the avoidance of doubt, the term independently means that where more than one substituent is selected from a number of possible substituents, those substituents may be the same or different.

As used herein the term "pharmaceutically acceptable derivative'' means any pharmaceutically acceptable salt, solvate, ester or amide, or salt or solvate of such ester or amide, of the compound of formula (I), or any other compound which upon administration to the recipient is capable of providing (directly or indirectly) the a compound of formula (I) or an active metabolite or residue thereof, e.g., a prodrug. Preferred pharmaceutically acceptable derivatives according to the invention are any pharmaceutically acceptable salts, solvates or prodrugs.

Suitable pharmaceutically acceptable salts of the compounds of formula (I) include acid salts, for example sodium, potassium, calcium, magnesium and tetraalkylammonium and the like, or mono- or di- basic salts with the appropriate acid for example organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids and the like. Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Hereinafter, compounds, their pharmaceutically acceptable salts, their solvates and polymorphs, defined in any aspect of the invention (except intermediate compounds in chemical processes) are referred to as "compounds of the invention".

The compounds of the invention may exist in one or more tautomeric forms. All tautomers and mixtures thereof are included in the scope of the present invention.

Compounds of the invention may exist in the form of optical isomers, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses.

Since the compounds of the invention are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis)- impure preparations of the compounds may be used for preparing the more pure forms used in the pharmaceutical compositions; these less pure preparations of the compounds should contain at least 1%, more suitably at least 5% and preferably from 10 to 59% of a compound of the invention.

Compounds of the invention may be prepared, in known manner in a variety of ways. In the following reaction schemes and hereafter, unless otherwise stated R¹ to R⁷, X and n are as defined in the first aspect. These processes form further aspects of the invention.

Throughout the specification, general formulae are designated by Roman numerals (I), (II), (III), (IV) etc. Subsets of these general formulae are defined as (Ia), (Ib), (Ic) etc .... (IVa), (IVb), (IVc) etc.

Compounds of formula (I) may be prepared from compounds of formula (II) according to reaction scheme 1, by reacting compounds of formula (II) with compounds of formula (III). Preferred reaction conditions comprise boron coupling of compounds of formula (III) where Y is -B(OH)₂ or 4,4,5,5-tetramethyl-[1,3,2]-dioxaborolan-2-yl cyclic derivative, with a compound of formula (II) in the presence of a suitable palladium catalysis (preferably Pd(PPh₃)₄) and a suitable base (preferably sodium carbonate) in an inert solvent (preferably 1,2-dimethoxyethane) at elevated temperature.

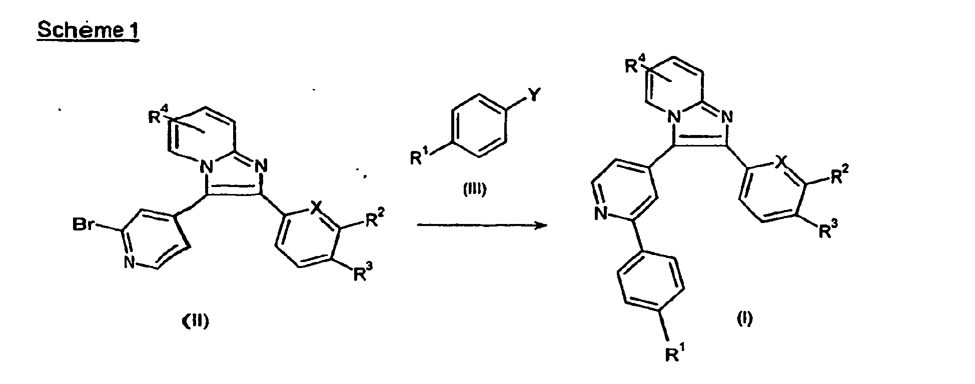

Compounds of formula (la), i.e. compounds of formula (I) where R¹ is -CH₂NR⁵R⁶, may be prepared by reductive amination of compounds of formula (IV) according to reaction scheme 2. Preferred reaction conditions comprise reacting (IV) with HNR⁵R⁶ in the presence of NaHB(OAc)₃, in a suitable solvent (preferably dichloromethane) at room temperature.

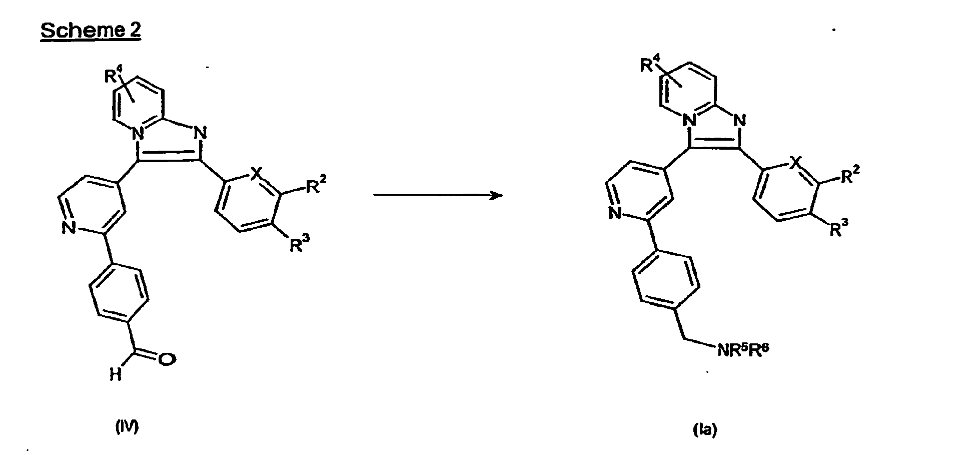

Compounds of formula (III) are available from commercial sources or may be prepared by methods analogous to those described in the Examples section hereinafter.

Compounds of formula (Ib), i.e. compounds of formula (I) where R¹ is -NR⁵R⁶, may be prepared according to reaction scheme 3 by reacting compounds of formula (1c), i.e. compounds of formula (I) where R¹ is bromine, with HNR⁵R⁶ in the presence of a catalyst system preferably tris(dibenzylideneacetone)dipalladium(O) and 2,2'-bis(diphenylphosphino)-1,1'-binaphthyl (Binap) in potassium tert-butoxide in a suitable solvent such as toluene at elevated temperature.

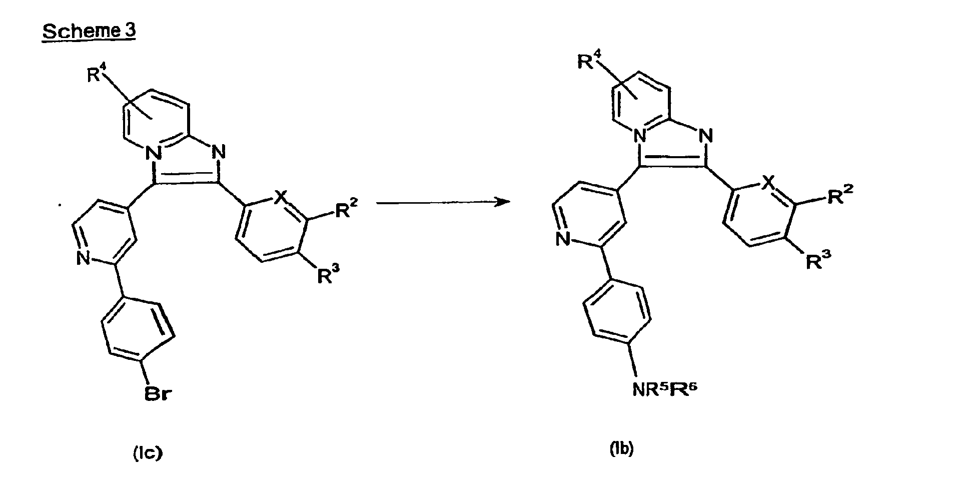

Compounds of formula (Id), i.e. compounds of formula (I) where R¹ is -OCH₂CH₂NR⁵R⁶, may be prepared according to reaction scheme 4 by reacting compounds of formula (V) with 1,2-dibromoethane in the presence of a base preferably potassium carbonate in a suitable solvent, such as acetone, at elevated temperature. Treatment with HNR⁵R⁶ in a suitable solvent such as tetrahydrofuran at elevated temperature gives (Id).

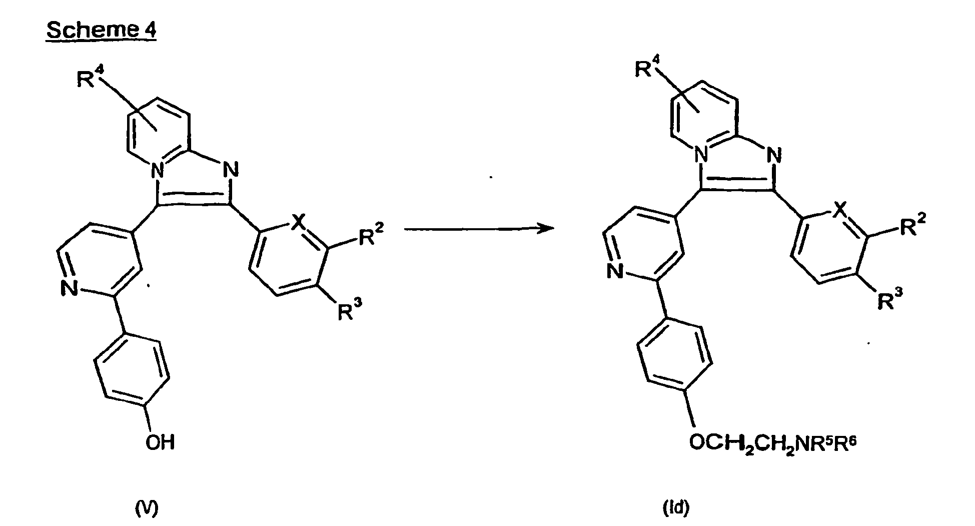

Compounds of formula (le), i.e. compounds of general formula (I) where R¹ is -CONR⁵R⁶, may be prepared according to reaction scheme 5. Compounds of formula (VI) (where R is methyl or ethyl) are firstly saponified by heating with sodium hydroxide in methanol, followed by conversion of the resulting carboxylic acid to amide (Ie). Preferred reaction conditions comprise treating the intermediate carboxylic acid with HNR⁵R⁶ in the presence of HOBT, EDCI and a suitable base such as triethylamine in a suitable solvent such as dimethylformamide at room temperature.

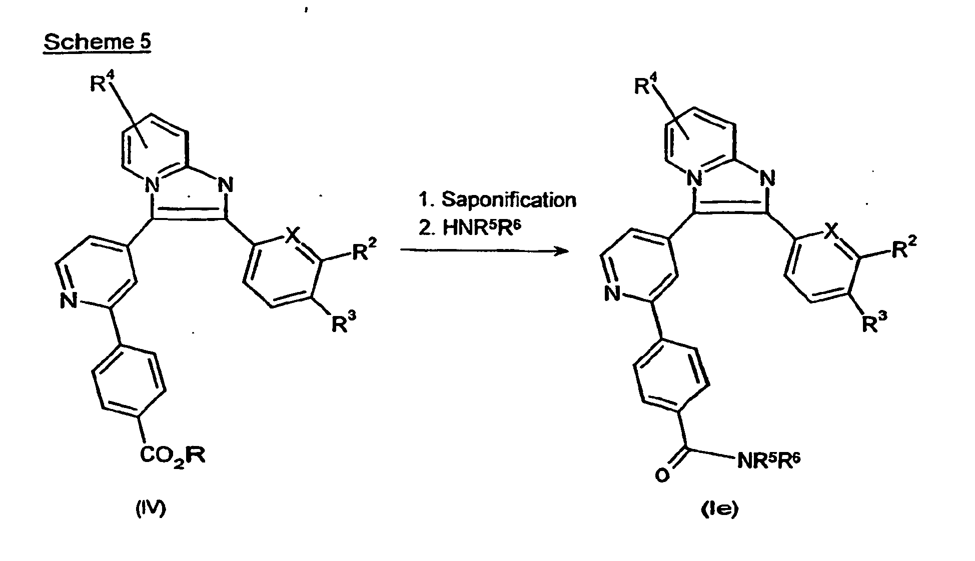

Compounds of formula (Ig), i.e. compounds of general formula (I) where R¹ is -NHSO₂CF₃, may be prepared in two steps according to reaction scheme 6. Firstly the acetyl group is removed from compounds of formula (Ih) by treatment with sodium hydroxide in methanol at elevated temperature. The resulting amine is then treated with CF₃SO₂Cl preferably in the presence of a base such as triethylamine in a suitable solvent such as dichloromethane at room temperature.

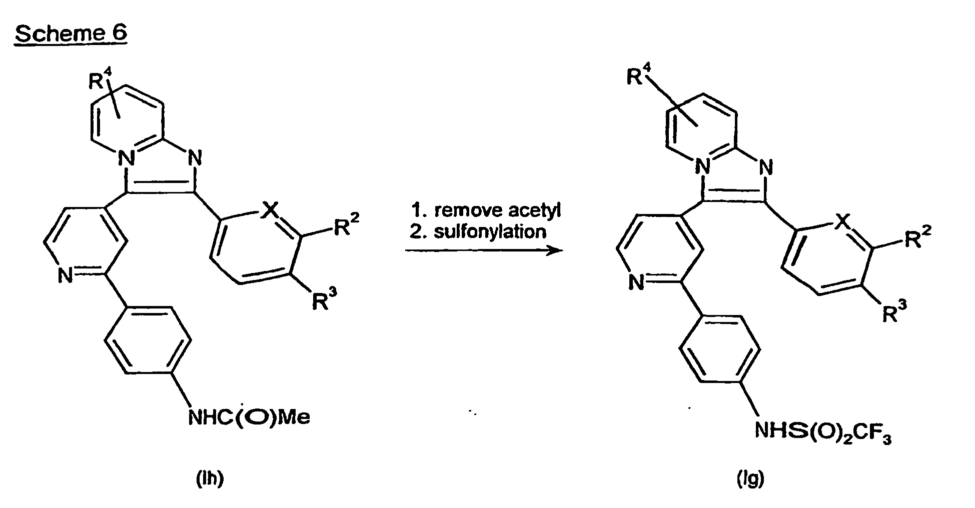

It will be apparent to the skilled person that compounds of formula (I) may also be prepared by introducing R¹ before formation of the imidazopyridine. For instance, compounds of formula (li), i.e. compounds of formula (I) where R¹ is morpholine, X is N and R³ is H may be prepared according to reaction scheme 7.

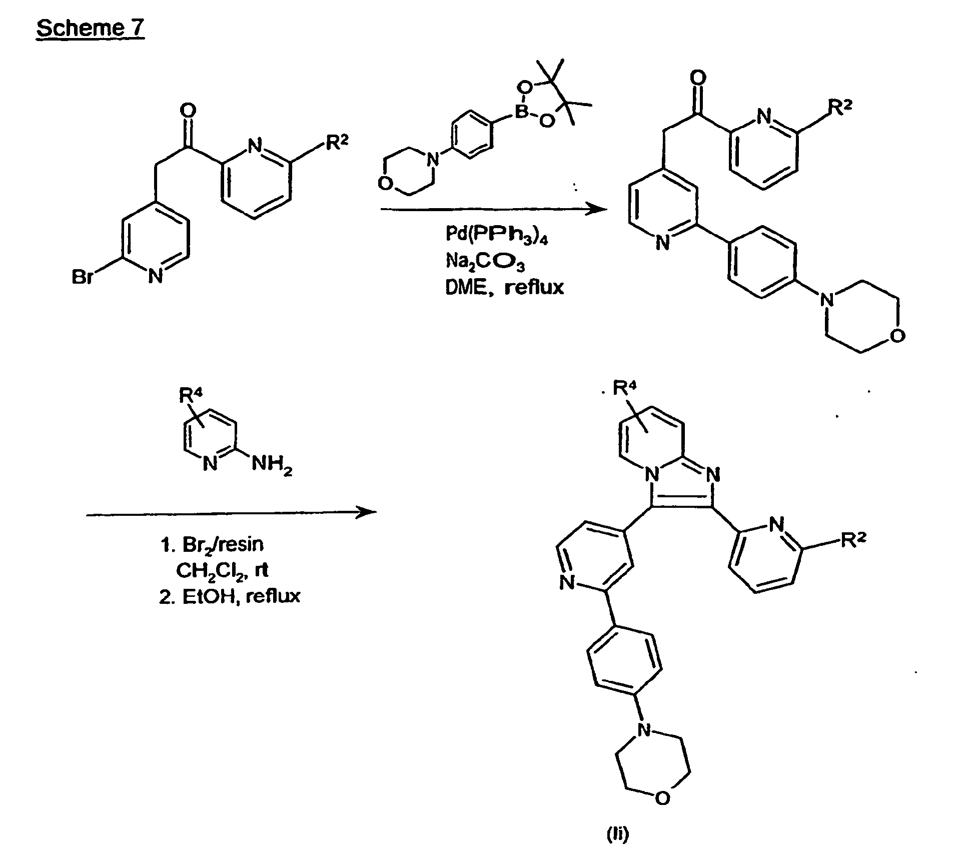

Compounds of formula (II) (see Scheme 1) may be prepared in two steps according to reaction scheme 8. Compounds of formula (VII) are firstly reacted with a suitable polymer-supported bromine reagent, such as polymer-supported pyridinium perbromide, in a suitable solvent such as dichloromethane at room temperature. Treatment with a compound of formula (VIII) in a suitable solvent such as ethanol at elevated temperature gives compounds of formula (II).

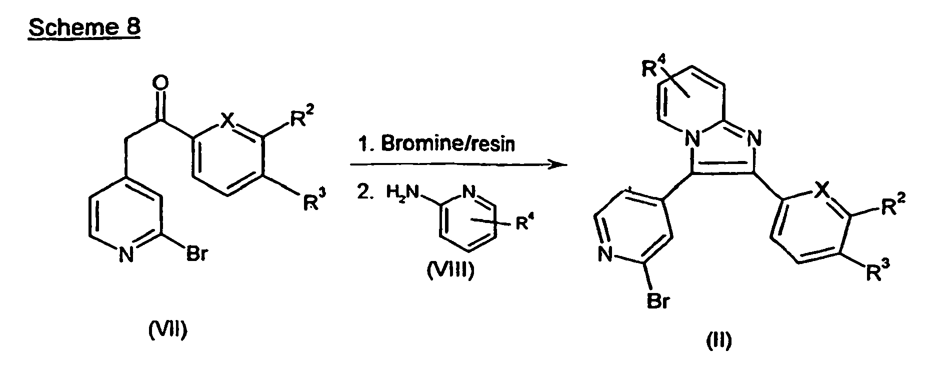

Compounds of formula (VII) may be prepared according to reaction scheme 9 by reacting 2-bromo-4-methylpyridine with compounds of formula (IX) in the presence of a suitable base such as sodium bis(trimethylsilyl)amide in a suitable solvent such as tetrahydrofuran at -78 °C to -30°C.

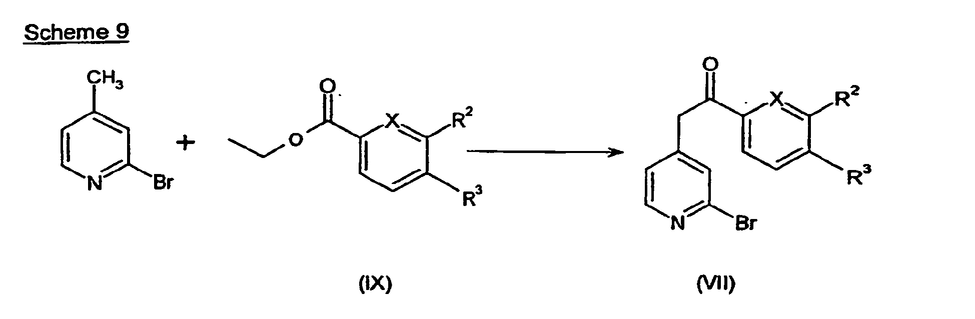

Further details for the preparation of compounds of formula (I) are found in the examples.

The compounds of the invention may be prepared singly or as compound libraries comprising at least 2, for example 5 to 1,000 compounds, and more preferably 10 to 100 compounds. Libraries of compounds of the invention may be prepared by a combinatorial 'split and mix' approach or by multiple parallel synthesis using either solution phase or solid phase chemistry, by procedures known to those skilled in the art. Thus according to a further aspect there is provided a compound library comprising at least 2 compounds of the invention.

Activation of the TGF-β1 axis and expansion of extracellular matrix are early and persistent contributors to the development and progression of chronic renal disease and vascular disease. Border W.A., *et al, N. Engl. J. Med*., 1994; 331(19), 1286-92. Further, TGF-β1 plays a role in the formation of fibronectin and plasminogen activator inhibitor-1. components of sclerotic deposits, through the action of smad3 phosphorylation by the TGF-β1 receptor ALK5. Zhang Y., *et al, Mature,* 1998; 394(6696), 909-13; Usui T., *et al, Invest. Ophthalmol. Vis. Sci*., 1998; 39(11), 1981-9.

Progressive fibrosis in the kidney and cardiovascular system is a major cause of suffering and death and an important contributor to the cost of health care. TGF-β1 has been implicated in many renal fibrotic disorders. Border W.A., *et al, N. Engl. J. Med*., 1994; 331(19), 1286-92. TGF-β1 is elevated in acute and chronic glomerulonephritis Yoshioka K., *et al, Lab. Invest.,* 1993; 68(2), 154-63, diabetic nephropathy Yamamoto, T., *et al*, 1993, *PNAS* 90, 1814-1818., allograft rejection, HIV nephropathy and angiotensin-induced nephropathy Border W.A., *et al, N. Engl. J. Med*., 1994; 331 (19), 1286-92. In these diseases the levels of TGF-β1 expression coincide with the production of extracellular matrix. Three lines of evidence suggest a causal relationship between TGF-β1 and the production of matrix. First, normal glomeruli, mesangial cells and non-renal cells can be induced to produce extracellular-matrix protein and inhibit protease activity by exogenous TGF-β1 in vitro. Second, neutralizing anti-bodies against TGF-β1 can prevent the accurnulation of extracellular matrix in nephritic rats. Third, TGF-β1 transgenic mice or in vivo transfection of the TGF-β1 gene into normal rat kidneys resulted in the rapid development of glomerulosclerosis. Kopp J.B., *et al, Lab. Invest,* 1996; 74(6), 991-1003. Thus, inhibition of TGF-β1 activity is indicated as a therapeutic intervention in chronic renal disease.

TGF-β1 and its receptors are increased in injured blood vessels and are indicated in neointima formation following balloon angioplasty Saltis J., *et al, Clin. Exp. Pharmacol. Physiol.,* 1996; 23(3),193-200. In addition TGF-β1 is a potent stimulator of smooth muscle cell ("SMC") migration in vitro and migration of SMC in the arterial wall is a contributing factor in the pathogenesis of atherosclerosis and restenosis. Moreover, in multivariate analysis of the endothelial cell products against total cholesterol, TGF-β receptor ALK5 correlated with total cholesterol (P < 0.001) Blann A.D., *et al, Atherosclerosis,* 1996; 120(1-2), 221-6. Furthermore, SMC derived from human atherosclerotic lesions have an increased ALK5/TGF-β type II receptor ratio. Because TGF-β1 is over-expressed in fibroproliferative vascular lesions, receptor-variant cells would be allowed to grow in a slow, but uncontrolled fashion, while overproducing extracellular matrix components McCaffrey TA, *et al*, Jr., *J*. *Clin. Invest*., 1995: 96(6), 2667-75. TGF-β1 was immunolocalized to non-foamy macrophages in atherosclerotic lesions where active matrix synthesis occurs, suggesting that non-foamy macrophages may participate in modulating matrix gene expression in atherosclerotic remodelling via a TGF-β-dependent mechanism. Therefore, inhibiting the action of TGF-β1 on ALK5 is also indicated in atherosclerosis and restenosis.

TGF-β is also indicated in wound repair. Neutralizing antibodies to TGF-β1 have been used in a number of models to illustrate that inhibition of TGF-β1 signalling is beneficial in restoring function after injury by limiting excessive scar formation during the healing process. For example, neutralizing antibodies to TGF-β1 and TGF-β2 reduced scar formation and improved the cytoarchitecture of the neodermis by reducing the number of monocytes and macrophages as well as decreasing dermal -fibronectin and collagen deposition in rats Shah M., *J*. *Cell. Sci.,* 1995,108, 985-1002. Moreover. TGF-β antibodies also improve heating of corneal wounds in rabbits Moller-Pedersen *T*., *Curr. Eye Res*., 1998. 17, 736-747, and accelerate wound heating of gastric ulcers in the rat, Ernst H., Gut, 1996,39, 172-175. These data strongly suggest that limiting the activity of TGF-β Would be beneficial in many tissues and suggest that any disease with chronic elevation of TGF-β would benefit by inhibiting smad2 and smad3 signalling pathways.

TGF-β is also implicated in peritoneal adhesions Saed G.M., *et al, Wound Repair Regeneration,* 1999 Nov-Dec, 7(6), 504-510. Therefore, inhibitors of ALK5 would be beneficial in preventing peritoneal and sub-dermal fibrotic adhesions following surgical procedures.

TGF-β is also implicated in photoaging of the skin (see Fisher GJ. Kang SW. Varani J. Bata-Csorgo Z. Wan YS. Data S. Voorhees JJ. , Mechanisms of photoaging and chronological skin ageing, *Archives of Dermatology*, 138(11):1462-1470, 2002 Nov. and Schwartz E. Sapadin AN. Kligman LH. "Ultraviolet B radiation increases steady state mRNA levels for cytokines and integrins in hairless mouse skin- modulation by topical tretinoin", Archives if Dermatological Research, 290(3):137-144, 1998 Mar.)

Therefore according to a further aspect, the invention provides the use of a compound defined in the first aspect in the preparation of a medicament for treating or preventing a disease or condition mediated by ALK-5 inhibition.

Preferably the disease or condition mediated by ALK-5 inhibition is selected from the list: chronic renal disease, acute renal disease, wound healing, arthritis, osteoporosis, kidney disease, congestive heart failure, ulcers (including diabetic ulcers, chronic ulcers, gastric ulcers, and duodenal ulcers), ocular disorders, comeal wounds, diabetic nephropathy, impaired neurological function, Alzheimer's disease, atherosclerosis, peritoneal and sub-dermal adhesion, any disease wherein fibrosis is a major component, including, but not limited to kidney fibrosis, lung fibrosis and liver fibrosis, for example, hepatitis B virus (HBV), hepatitis C virus (HCV), alcohol-induced hepatitis, haemochromatosis, primary biliary cirrhosis, restenosis, retroperitoneal fibrosis, mesenteric fibrosis, endometriosis, keloids, cancer, abnormal bone function, inflammatory disorders, scarring and photaging of the skin.

More preferably the disease or condition mediated by ALK-5 inhibition is fibrosis. Preferably kidney fibrosis.

It will be appreciated that references herein to treatment extend to prophylaxis as well as the treatment of established conditions.

Compounds of the invention may be administered in combination with other therapeutic agents, for example antiviral agents for liver diseases, or in combination with ACE inhibitors or angiotensin II receptor antagonists for kidney diseases.

The compounds of the invention may be administered in conventional dosage forms prepared by combining a compound of the invention with standard pharmaceutical carriers or diluents according to conventional procedures well known in the art.
These procedures may involve mixing, granulating and compressing or dissolving the ingredients as appropriate to the desired preparation.

The pharmaceutical compositions of the invention may be formulated for administration by any route, and include those in a form adapted for oral, topical or parenteral administration to mammals including humans.

The compositions may be formulated for administration by any route. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

It will be recognised by one of skill in the art that the optimal quantity and spacing of individual dosages of a compound of the invention will be determined by the nature and extent of the condition being treated, the form, route and site of administration, and the particular mammal being treated, and that such optimums can be determined by conventional techniques. It will also be appreciated by one of skill in the art that the optimal course of treatment, i.e., the number of doses of a compound of the invention given per day for a defined number of days, can be ascertained by those skilled in the art using conventional course of treatment determination tests.

No toxicological effects are indicated when a compound of the invention is administered in the above-mentioned dosage range.

All publications, including, but not limited to, patents and patent applications cited in this specification, are herein incorporated by reference as if each individual publication were specifically and individually indicated to be incorporated by reference herein as though fully set forth:

It will be appreciated that the invention includes the following further aspects. The preferred embodiments described for the first aspect extend these further aspects:
i) a pharmaceutical composition comprising a compound of the invention and a pharmaceutically acceptable carrier or diluent;
ii) a compound of the invention for use as a medicament;
iii) a method of treatment or prophylaxis of a disorder selected from chronic renal disease, acute renal disease, wound healing, arthritis, osteoporosis, kidney disease, congestive heart failure, ulcers (including diabetic ulcers, chronic ulcers, gastric ulcers, and duodenal ulcers), ocular disorders, comeal wounds, diabetic nephropathy, impaired neurological function, Alzheimer's disease, atherosclerosis, peritoneal and sub-dermal adhesion, any disease wherein fibrosis is a major component, including, but not limited to kidney fibrosis, lung fibrosis and liver fibrosis, for example, hepatitis B virus (HBV), hepatitis C virus (HCV), alcohol-induced hepatitis, haemochromatosis, primary biliary cirrhosis, restenosis, retroperitoneal fibrosis, mesenteric fibrosis, endometriosis, keloids, cancer, abnormal bone function, inflammatory disorders, scarring and photoaging of the skin, in mammals, which comprises administration to the mammal in need of such treatment, an effective amount of a compound of the invention; and
iv) a combination of a compound of the invention with an ACE inhibitor or an angiotensin II receptor antagonist.

According to the invention there is provided a compound of formula (I) or a pharmaceutically acceptable salt, solvate or derivative thereof: 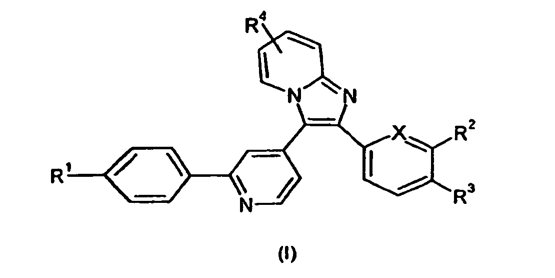 wherein X is N or CH;
R¹ is selected from H, C₁₋₆alkyl, C₁₋₆alkenyl, C₁₋₆alkoxy, halo, cyano, perfluoro C₁₋₆ alkyl, perfluoroC₁₋₆alkoxy, -NR⁵R⁶, -(CH₂)ₙR⁵R⁶, -O(CH₂)ₙOR⁷, -O(CH₂)ₙNR⁵R⁶, -CONR⁵R⁶, -CO(CH₂)ₙNR⁵R⁶, -SO₂R⁷, -SO₂NR⁵R⁶, -NR⁵SO₂R⁷ and -NR⁵COR⁷;
R² is selected from H, C₁₋₆alkyl, halo, CN or perfluoroC₁₋₆alkyl;
R³ is selected from H or halo;
R⁴ is selected from H, halo, C₁₋₆alkyl or -NR⁵R⁶,
R⁵, R⁶ and R⁷ are independently selected from H or C₁₋₆alkyl; or R⁵ and R⁶ together with the atom to which they are attached form a 3,4, 5, 6 or 7-membered saturated or unsaturated ring which may contain one or more heteroatoms selected from N, S or O, and wherein the ring may be further substituted by one or more substituents selected from halo (such as fluoro, chloro, bromo), - CN, -CF₃, -OH, -OCF₃, C₁₋₆ alkyl and C₁₋₆ alkoxy; and
n is 1-4.

The following non-limiting examples illustrate the present invention.

### Abbreviations

| | | |
|---|---|---|
| Binap | - | 2,2'-bis(diphenylphosphino)-1,1'-binaphthyl |
| CH₂Cl₂ | - | dichloromethane |
| DME | - | 1,2-Dirnethoxyethane |
| DMF | - | dimethylformamide |
| EDCl | - | 1-(3-dimethylaminopropyl)-3-ethylcarbodiimide hydrochloride |
| EtOH | - | ethanol |
| EtOAc | - | ethyl acetate |
| HOBT | - | 1-hydroxybenzotriazole hydrate |
| KMnO₄ | - | potassium permanganate |
| NaHB(OAc)₃ | - | sodium triacetoxyborohydride |
| NaHMDS | - | sodium bis(trimethylsilyl)amide |
| NaOH | - | sodium hydroxide |
| Na₂SO₄ | - | sodium sulfate |
| MeOH | - | methanol |
| THF | - | tetrahydrofuran |
| TEA | - | triethylamine |
| DME | - | dimethyoxyethane |
| Pd₂(dba)₃ | - | tris (dibenrylideneacetone)dipalladium |
| Pd(PPh₃)₄ | - | tetrakis(triphenlyphosphine) palladium(0) |
| PTS | - | para-toluene sulfonic acid |

### Intermediate 1: 3-Chloro-4-fluoro-benzoic acid ethyl ester

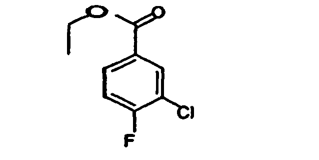

To a solution of 3-chloro-4-fluoro-benzoic acid (11.75 g, 67.3 mmol) in EtOH was added PTS (1.2 g) and the resulting mixture was heated under reflux for 2 days. On cooling the mixture was poured into water. The aqueous phase was basified with a solution of NaOH 1N. The product was extracted with CH₂Cl₂ and the organic phase was dried over Na₂SO₄ and concentrated under reduced pressure to give the title compound as an oil (13.08g, 96%); [APCI MS] m/z 203 (MH+).

### Intermediate 2: 3.4-Difluoro-benzoic acid ethyl ester

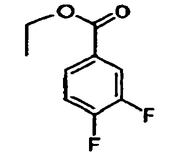

3,4-Difluoro-benzoic acid (11 g, 69.57 mmol) was reacted as described for intermediate 1 to afford the title compound as an oil (11.78g, 91%); ¹H NMR (300 MHz, CDCl₃) δ ppm: 7.84 (m, 2H), 7.22 (m, 1H), 4.37 (q, 2H), 1.38 (t, 3H).

### Intermediate 3: 6-Methyl-pyridine-2-carboxylic acid ethyl ester

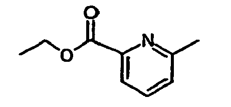

6-Methyl-pyridine-2-carboxylic acid (25g, 182.3 mmol) was reacted as described for intermediate 1 to afford the title compound as an oil (22.9g, 76.13%); ¹H NMR (300 MHz, CDCl₃) δ ppm: 7.95 (d, 1H), 7.75 (t, 1H), 7.35 (d, 1H), 4.5 (q, 2H), 2.7 (s, 3H), 1.45 (t, 3H).

### Intermediate 4: 6-Fuoro-pyridine-2-carboxylic acid

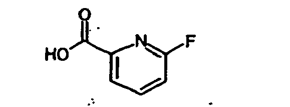

To a solution of 2-fluoro-6-methyl-pyridine (2.5g, 22.5 mmol) in water (170 ml) was added portion-wise KMnO₄(2g, 12.65 mmol) and the mixture was heated to reflux. KMnO₄ (8g, 50.63 mmol) was added portion-wise and the mixture was heated under reflux for 3 hours and then cooled. The precipitate was filtered and the filtrate was acidified with a solution of HCl and then concentrated under reduced pressure. The residue was triturated with hot EtOH, the solid filtered and the filtrate was concentrated to dryness under reduced pressure. The title compound was obtained as a white solid (1.7g, 53%) ; m.p. 137°C.

### Intermediate 5: 6-Fluoro-pyridine-2-carboxylic acid isopropyl ester

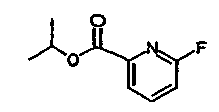

Intermediate 4 (1g, 7.09 mmol) was added portion-wise to thionyl chloride (3 ml) and the mixture was heated under reflux for 3 hours and then concentrated under reduced pressure. Isopropanol (3 ml) was added to the residue and the mixture was stirred at room temperature for 5 minutes and then concentrated under reduced pressure. The residue was treated with a saturated aqueous solution of NaHCO₃ and extracted with ethyl acetate. The combined organic phases were dried over Na₂SO₄ and concentrated under reduced pressure. The title compound was obtained as an oil (1.2g, 93%); [APCI MS] m/z 184 (MH+).

### Intermediate 6: 1-Methyl-4-hydroxymethyl-imidazole

To a suspension of 1-methyl-imidazole-4-carboxylic acid (11.4g, 90 mmol) in THF (500ml) at 0°C, was added drop-wise a solution of lithium aluminium hydride (1 M in THF, 117ml, 117 mmol) and the mixture was stirred at room temperature overnight and then at 50°C for 1 hour. Water (3 ml) was added followed by Na₂SO₄ and the mixture was filtered through celite™. The filtrate was concentrated under reduced pressure to afford the title compound as a solid (8g, 78.95%); ¹H NMR (300 MHz, CDCl₃) δ ppm: 7.25 (s, 1H), 6.7 (s, 1H), 5.25 (m, 1H), 4.4 (s, 2H), 3.45 (s, 3H).

### Intermediate 7: 1-Methyl-4-chloromethyl-imidazole hydrochloride

To a solution of intermediate 6 (5g, 44.64 mmol) in CH₂Cl₂ (10 ml) at 0°C was added dropwise thionyl chloride (50 ml) and the mixture was stirred at room temperature overnight and then at reflux for 3 hours. The mixture was concentrated under reduced pressure and the residue taken up in diethyl ether to give a precipitate. The precipitate was filtered and dried to give the title compound (4g, 53.81%); ¹H NMR (300 MHz, DMSO- d₆) δ ppm: 9.25 (s, 1H), 7.8 (s, 1H), 4.95 (s, 2H), 3.9 (s, 3H).

### Intermediate 8: 4-(Morpholin-4-yl)-bromobenzene

To an ice-cooled solution of 4-phenyl-morpholine (18g, 110.4 mmol) in ethanol (400ml), was added dropwise bromine (5.95 ml, 115.9 mmol). After addition the mixture was warmed to room temperature and stirred for 2 hours. The mixture was poured into water and the solution was basified with 1 N sodium hydroxide solution. The resulting precipitate was filtered, washed with water and dried. Recrystallisation from diisopropyl ether gave the title compound as white crystals (15g, 56.13%); m.p. 126-128°C.

### Intermediate 9 : N-[4-(4,4,5,5-Tetramethyl-[1,3,2]-dioxaborolan-2-yl)-phenyl]-morpholine

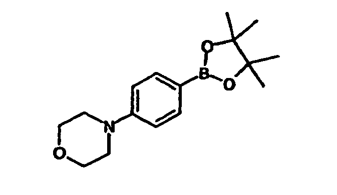

To a solution of intermediate 8 (20g, 82.64 mmol) in dioxane (200 ml) was added 4,4,5,5-tetramethyl-[1,3,2]-dioxaborolane (13.2 ml, 99.17 mmol), dichloro bis(triphenylphosphine) palladium (II) (3g, 4.13 mmol) and triethylamine (34.5 ml, 247.93 mmol) and the mixture was heated under reflux for 4 hours. The reaction mixture was cooled, poured into water and extracted with CH₂Cl₂. The combined organic phases were dried over Na₂SO₄ and concentrated under reduced pressure. The residue was purified by chromatography on silica gel eluting with CH₂Cl₂ to give the title compound was obtained as an orange oil which crystallised (19.98 g, 83.94%); [APCI MS] m/z 289.07 (MH⁺).

### Intermediate 10: 4-(4,4,5,5-Tetramethyl-[1,3,2]dioxaborolan-2-yl)-N-(tetrahydropyran-4-yl)-benzamide

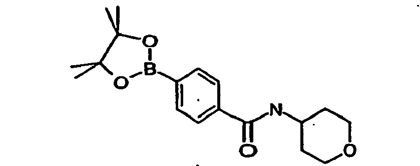

4-(4,4,5,5-Tetramethyl-[1,3,2]dioxaborolan-2-yl)-benzoic acid (70.16g, 0.28 mol) was treated with thionyl chloride (2 vol) and the reaction mixture was stirred at reflux for 2 hours. The mixture was cooled and evaporated to give a residue. The residue was dissolved in toluene and the mixture was poured into a solution of tetrahydro-pyran-4-ylamine (34.34g, 0.339) and triethylamine (79 mL, 0.57 mol) in CH₂Cl₂ at 10°C. The mixture was warmed to room temperature and stirred for 2 days. Addition of water (490 ml) gave a precipitate which was filtered and washed with EtOAc. Purification by flash chromatography eluting with CH₂Cl₂/MeOH (95:5) gave the title compound as a solid (17.02g, 18%); ¹H NMR (400 MHz, CDCl₃) δ ppm: 7.85 (d, 2H), 7.72 (d, 2H), 5.98 (m, 1H), 4.20 (s, 1H), 3.99 (m, 2H), 3.35 (t, 2H), 2.01 (d, 2H), 1.57 (m, 2H), 1.35 (s, 12H).

### Intermediate 11: N-[4-(4,4,5,5-tetramethyl-[1,3,2]dioxaborolan-2-yl)-phenyl]-methanesulfonamide

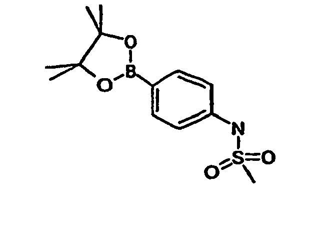

To a solution of 4-(4,4,5,5-tetramethyl-[1,3,2]dioxaborolan-2-yl)-aniline (5g, 22.8 mmol) in CH₂Cl₂ (20ml) was added NaHCO₃ (2.3g, 27.4 mmol) and methanesulfonyl chloride (13.2 mL, 171 mmol) and the reaction mixture was stirred at room temperature for 6 days. Water was added and the mixture was extracted with CH₂Cl₂. The organic phase was dried over Na₂SO₄, filtered and concentrated under reduced pressure. The residue was recrystallised from diethyl ether to give the title compound as a white powder (2.52g, 37%); ¹H NMR (300 MHz, CDCl₃) δ ppm: 7.78 (d, 2H), 7.18 (d, 2H), 6.69 (m, 1H), 3.02 (s, 3H), 1.33 (s, 12H).

### Intermediate 12: N-[(4-(4,4,5,5-tetramethyl-[1,3,2]dioxaborolan-2-yl)-phenyl)carbonyl]-morpholine

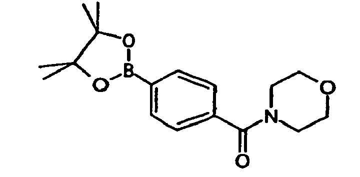

To a solution of 4-(4,4,5,5-tetramethyl-[1,3,2]dioxaborolan-2-yl)-benzoic acid (5g, 20.15 mmol) in CH₂Cl₂/DMF (50ml/5ml) was added morpholine (2.1 ml, 24.2mmol), HOBT (3.3g, 24.2mmol), EDCI (4.65g, 24.2mmol) and triethylamine (4.2ml, 30.2mmol) and the reaction mixture was stirred at room temperature for 3 days. Water was added and the product was extracted with CH₂Cl₂. The combined organic extracts were dried over Na₂SO₄ and concentrated under reduced pressure to give a reside. Trituration of the residue with diisopropyl ether gave the title product as a white solid (4.21g, 66%); ¹H NMR (300 MHz, CDCl₃) δ ppm: 7.8 (d, 2H), 7.4 (d, 2H), 3.7 (m, 4H), 3.55 (m, 2H), 3.35 (m, 2H), 1.3 (s, 12H).

### Intermediate 13: 1-Ethyl-4-[(4-(4,4,5,5-tetramethyl-[1,3,2]dioxaborolan-2-yl)-phenyl)carbonyl]-piperazine

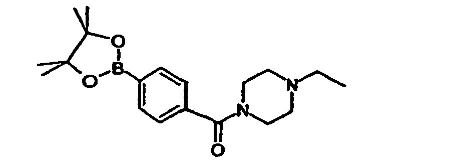

4-(4,4.5.5-Tetramethyl-[1,3,2]dioxaborolan-2-yl)benzoic acid (8.24g, 33.22 mmol) and N-ethylpiperazine (5.1 ml, 39.87mmol) were reacted as described for intermediate 12 to give, after chromatography on silica gel (CH₂CI₂/MeOH, 95:5), the title compound (9.64g, 84%); [APCI MS] m/z 345 (MH⁺).

### Interrnediate 14: 2-[2-Bromo-pyridin-4-yl]-1-pyridin-2-yl-ethanone

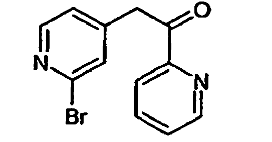

To a solution of 2-bromo-4-methyl-pyridine (27 g. 157 mmol) in dry THF (270 ml) was added ethyl picolinate (28.5 g, 188.7 mmol). The resulting mixture was cooled to -78°C under argon and a solution of sodium bis-(trimethylsilyl)amide (1M in THF, 345 ml, 345 mmol) was added dropwise at -78°C. The reaction mixture was allowed to reach room temperature and stirred overnight. The solvent was evaporated under reduced pressure and the residue triturated with diethyl ether. The solid was filtered and washed with diethyl ether. The solid was taken-up in saturated NH₄Cl solution and the aqueous phase extracted with ethyl acetate. The organic phase was dried over Na₂SO₄ and concentrated to give an orange powder which was washed with pentane to give the title compound as a yellow solid (33.97 g); m.p. 111.2°C.

### Intermediate 15: 2-[2-Bromo-pyridin-4-yl]-1-(6-methyl-pyridin-2-yl)-ethanone

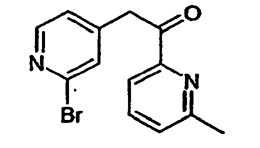

A solution of sodium bis-(trimethylsilyl)amide (2M in THF, 32 ml; 64 mmol) was added dropwise at -30°C to a solution of 2-bromo-4-methyl-pyridine (5 g, 29mmol) in dry THF (70 ml). The mixture was stirred at -30°C for 1 hour and then 6-methylpicolinic acid methyl ester (4.82 g, 32.3mmol, 1.1eq) was added. The reaction mixture was stirred at room temperature overnight. Diethyl ether was added and the resulting solid was filtered and washed with diethyl ether. The solid was taken up in saturated NH₄Cl solution and the aqueous phase was extracted with ethyl acetate. The organic layer was dried over Na₂SO₄ and concentrated. The resulting orange powder was washed with pentane to give the title compound as a yellow solid (5.84g, 70%); [APCI MS] m/z 292 (MH+).

### Intermediate 16 : 2-(2-Bromo-pyridin-4-yl)-1-(3-chloro-4-fluoro-phenyl)-ethanone

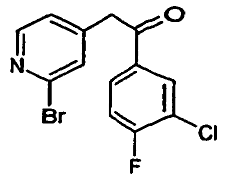

2-Bromo-4-methyl-pyridine (9.2g, 53.5 mmol) and 3-chloro-4-fluoro-benzoic acid ethyl ester (13 g, 64.2 mmol) were reacted as described for intermediate 14 to afford the title compound as an orange solid (17.16 g, 98%); [APCI MS] m/z: 330 (MH+).

### Intermediate 17: 2-(2-Bromo-pyridin-4-yl)-1-(3,4-difluoro-phenyl)-ethanone

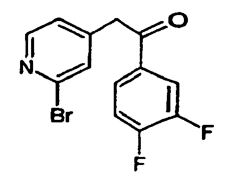

2-Bromo-4-methyl-pyridine (9.056g , 52.64 mmol) and 3,4-difluoro-benzoic acid ethyl ester (11.75 g, 63.17 mmol) were reacted as described for intermediate 14 to afford the title compound as an ocre solid (14.54 g, 88.5%); [APCI MS] m/z: 314 (MH+).

### Intermediate 18: 2-(2-Bromopyridin-4-yl)-1-(3-chloro-phenyl)-ethanone

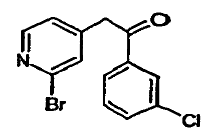

2-Bromo-4-methyl-pyridine (7.75g , 45.1 mmol) and methyl-3-chlorobenzoate (10 g, 58.6 mmol) were reacted as described for intermediate 14 to afford the title compound as an orange powder (13.02 g, 93%); ¹H NMR (300 MHz, CDCl₃) δ ppm: 8.34 (d, 1H), 7.95 (m, 1H), 7.84 (d, 1H), 7.59 (d, 1H), 7.46 (d, 1H), 7.41 (d, 1H), 7.13 (d, 1H), 4.24 (s, 2H).

### Intermediate 19: 2-(2-Bromo-pyridin-4-yl)-1-(6-fluoro-pyridin-2-yl)-ethanone

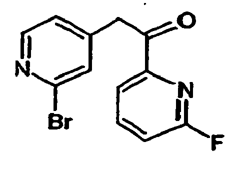

To a solution of 2-bromo-4-methyl-pyridine (2.58g, 15 mmol) in anhydrous THF (50 ml) at -30°C, was added dropwise NaHMDS (solution 2M in THF, 15ml, 30 mmol) and the mixture was stirred at -30°C for 2 hours. A solution of intermediate 5 (2.74g, 15 mmol) in THF (50 ml) was added dropwise and the mixture was stirred at -30°C for 1 hour. The mixture was allowed to reach room temperature and poured into water. The mixture was extracted with EtOAc, the combined organic phases were dried over Na₂SO₄ and the mixture was concentrated under reduced pressure. The concentrate was purified by chromatography on silica gel (CH₂Cl₂/MeOH, 99:1) to give the title compound as a yellow solid (1.6g, 36%); [APCI MS] m/z 295 (MH⁺).

### Intermediate 20: 2-(2-(4-(Morpholin-4-yl)phenyl)-pyridin-4-yl)-1-(6-methyl-pyridin-2-yl)-ethanone

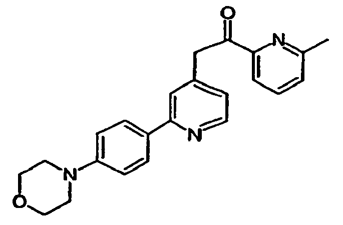

To a solution of intermediate 15 (3g, 10.3 mmol) in DME (100 mL) was added tetrakis(triphenylphosphine)palladium(0) (1.2g , 10%mol), intermediate 9 (3.86g, 13.4 mmol) and Na₂CO₃ (2M, 10.3 ml) and the mixture was heated under reflux for 18 hours. The cooled mixture was poured into water and extracted with CH₂Cl₂. The organic phase was washed with water, dried over Na₂SO₄ and filtered. Evaporation of the solvent *in vacuo* gave a crude oil which was purified by chromatography on silica gel (CH₂CI₂/MeOH, 97:3) to give the title compound (3.77g, 98%); [APCI MS] m/z 374.13 (MH+).

### Intermediate 21: 3-(2-Bromo-pyridin-4-yl)-2-(pyridin-2-yl)-imidazo[1,2-a]pyridine

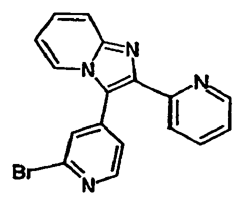

To a solution of intermediate 14 (5g, 18.05mmol) in CH₂Cl₂ (30 ml) was added bromine-polymer-supported (11.28g, 18:05 mmol) and the suspension was stirred at room temperature for 5 hours. The suspension was filtered, washing through ethanol. The filtrate and washings were added to 2-aminopyridine (3.4 g , 36.06 mmol) and the mixture heated under reflux for 18 hours. On cooling the mixture was concentrated and the residue was extracted between water and CH₂Cl₂. The combined organic phases were dried over Na₂SO₄ and evaporated under reduced pressure to give a crude solid which was precipitated from diisopropyl ether to afford the title compound (3.053g; 48%); m.p. 227°C.

### Intermediate 22: 3-(2-Bromo-pyridin-4-yl)-2-(6-methyl-pyridin-2-yl)-imidazo[1,2-a]pyridine

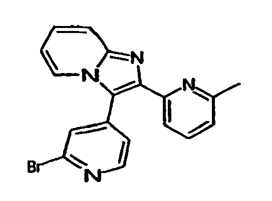

Intermediate 15 (5g, 17.18mmol) and 2-aminopyridine (3.23g, 34.32mmol) were coupled and treated as described for intermediate 21 to afford the title compound as a brown powder (3.621g, 58%); ¹H NMR (300 MHz, CDCl₃) δ ppm: 8.47 (d, 1H); 8.12 (d, 1H); 7.8 (m, 2H); 7.7 (d, 1 H); 7.6 (t, 1H); 7.47 (d, 1H); 7.29 (t, 1 H); 7.05 (d, 1H); 6.85 (t, 1H) ; 2.39 (s, 3H).

### Intermediate 23: 3-(2-Bromo-pyridin-4-yl)-2-(3-chloro-4-fluoro-phenyl)-imidazo[1,2-a]pyridine

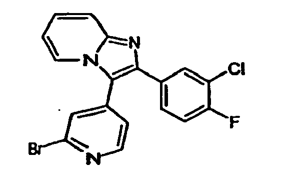

Intermediate 16 (5g, 15.22 mmol) and 2-amino-pyridine (2.86g , 30.44mmol) were coupled and treated as described for intermediate 21 to afford the title compound (3g, 49%); [APCI MS] m/z 404 (MH+).

### Intermediate 24: 3-(2-Bromo-pyridin-4-yl)-2-(3,4-difluoro-phenyl)-imidazo[1,2-a]pyridine

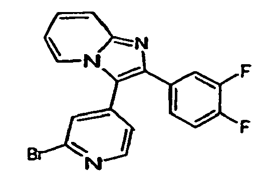

Intermediate 17 (5g, 16 mmol) and 2-aminopyridine (3g, 32 mmol) were coupled and treated as described for intermediate 21 to afford, after crystallisation from diisopropyl ether, the title compound as a brown powder (2.95g, 88%); [APCI MS] m/z 386 (MH+).

### Intermediate 25: 3-(2-Bromo-pyridin-4-yl)-6-chloro-2-(6-methyl-pyridin-2-yl)-imidazo[1,2-a]pyridine

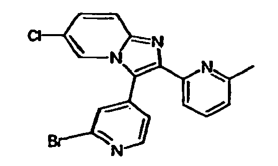

Intermediate 15 (2g, 6.87 mmol) and 2-amino-5-chloropyridine (1.77g, 13.75 mmol) were coupled and treated as described for intermediate 21 to afford the title compound as a brown powder (1.152g, 42%); ¹H NMR (300 MHz, CDCl₃) δ ppm: 8.50 (d, 1H), 8.09 (d, 1 H), 7.82 (s, 2H), 7.65 (t, 2H), 7.45 (d, 1H), 7.27 (d, 1 H), 7.08 (d, 1 H), 2.39 (s, 3H).

### Intermediate 26: 3-(2-Bromo-pyridin-4-yl)-7-methyl-2-(6-methyl-pyridin-2-yl)-imidazo[1,2-a]pyridine

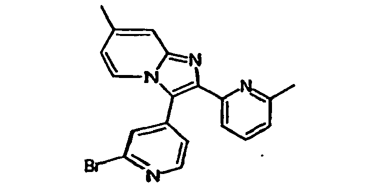

Intermediate 15 (2.53g, 6.87 mmol) and 2-amino-4-picoline (1.49g, 13.75 mmol) were reacted and treated as described for intermediate 21 to afford the title compound as a brown solid (1.43g, 55%); ¹H NMR (300 MHz, CDCl₃) δ ppm: 8.43 (d, 1H), 8.00 (d, 1H), 7.82 (s, 1H), 7.78 (d, 1H), 7.60 (t. 1H), 7.44 (m, 2H), 7.05 (d, 1 H), 6.70 (d, 1H), 2.43 (s, 3H), 2.40 (s, 3H).

### Intermediate 27: 3-(2-Bromo-pyridin-4-yl)-8-methyl-2-(6-methyl-pyridin-2-yl)-imidazo[1,2-a]pyridine

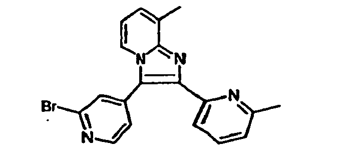

Intermediate 15 (5g, 17.24 mmol) and 2-amino-3-picoline (3.73g, 34.5 mmol) were coupled and treated as described for intermediate 21 to afford the title compound as a brown solid (4.08g, 62%); ¹H NMR (300 MHz, CDCl₃) δ ppm: 8.55 (d, 1H), 8 (d, 1H), 7.85 (d, 1H), 7.8 (s, 1H), 7.65 (t, 1H), 7.45 (d, 1 H), 7.1 (m, 2H), 6.8 (t, 1H), 2.7 (s, 3H), 2.4 (s, 3H).

### Intermediate 28 : 3-(2-Bromo-pyridin-4-yl)-2-(3-chloro-phenyl)-imidazo[1,2-a]pyridine

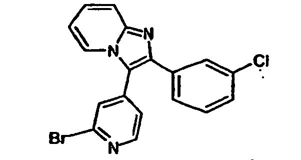

Intermediate 18 (13g, 42.1 mmol) and 2-amino-pyridine (7.9g, 2eq, 84 mmol) were coupled and treated as described for intermediate 21 to afford the title compound as a yellow solid (8.45g, 52%); [APCI MS] m/z 384 (MH+).

### Intermediate 29: 3-(2-Bromo-pyridin-4-yl)-2-(6-fluoro-pyridin-2-yl)-imidazo[1,2-a]pyridine

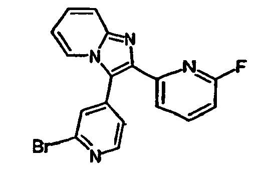

Intermediate 19 (0.65g, 2.1 mmol) and 2-aminopyridine (0.42g, 4.4 mmol) were coupled and treated as desaibed for intermediate 21 to afford, after trituration with diisopropyl ether, the title compound as a cream solid (199mg, 25%); [APCI MS] m/z 369 (MH+).

### Intermediate 30: 3-(2-(4-Formyl-phenyl)-pyridin-4-yl)-2-(6-methyl-pyridin-2-yl)-imidazo[1,2-a]pyridine

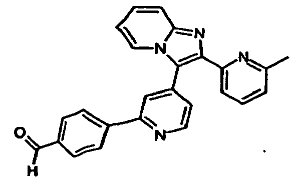

A solution of intermediate 22 (500mg, 1.37 mmol) in DME (50 ml) was treated with tetrakis(triphenylphosphine)palladium(O) (158 mg, 10%mol) and stirred at room temperature for 30 min. Aqueous Na₂CO₃ (2M, 4.2 ml) was added to the reaction mixture, followed by 4-formylphenyl boronic acid (267mg, 1.78 mmol) and the mixture was heated under reflux overnight. The cooled mixture was poured into ice and extracted with CH₂Cl₂. The organic phase was washed with water, dried over Na₂SO₄ and filtered. Evaporation of the solvent *in vacuo* gave a crude oil which was purified by chromatography on silica gel (CH₂Cl₂/MeOH 95:5) to give the title compound (310 mg, 58%); ¹H NMR (300 MHz, CDCl₃) δ ppm: 10.08 (s, 1 H) ; 8.86 (d, 1H); 8.10-8.20 (m, 4H); 7.98 (d, 1H); 7.83 (d, 1H); 7.75 (d, 1H); 7.61 (t, 1H); 7.51 (m, 1H); 7.30 (t, 1H); 7.04 (d, 1H) ; 6.85(t, 1H) ; 2.31 (s, 3H).

### Intermediate 31: 3-(2-(4-Formyl-phenyl)-pyridin-4-yl)-2-pyridin-2-yl-imidazo[1,2-a]pyridine

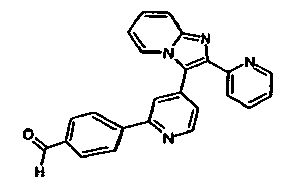

Intermediate 21 (1.2g, 3.4mmol) and 4-formylphenyl boronic acid (612mg. 4.1 mmol) were coupled and treated as described for intermediate 30 to afford after crystallisation from acetonitrile, the compound as a cream powder (1.1g, 86%); m.p. 216-218°C; [APCI MS] m/z 377 (MH+).

### Intermediate 32: 3-(2-(4-Formyl-phenyl)-pyridin-4-yl)-2-(3-chloro-4-fluoro-phenyl)-imidazo[1,2-a]pyridine

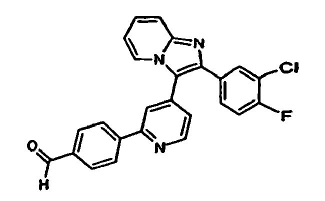

Intermediate 23 (1g, 2.48 mmol) and 4-formylphenyl boronic acid (484mg, 3.22 mmol) were coupled and treated as described for intermediate 30 to afford, after purification by chromatography on silica gel (CH₂CI₂/MeOH 98:2), the title compound as a yellow solid (380 mg, 36%); [APCI MS] m/z 428 (MH+).

### Intermediate 33: 3-(2-(4-Formyl-phenyl)-pyridine-4-yl)-2-(3,4-difluoro-phenyl)-imidazo[1,2-a]pyridine

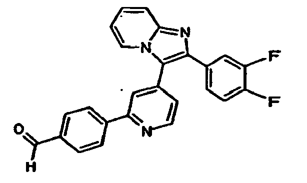

Intermediate 24 (1g, 2.6 mmol) and 4-formylphenyl boronic acid (506mg, 3.37 mmol) were coupled and treated as described for intermediate 30 to afford the title compound as a solid (600 mg, 56%); [APCI MS] m/z 412 (MH+).

### Intermediate 34: 6-Chloro-3-(2-(4-formyl-phenyl)-pyridin-4-yl)-2-(6-methyl-pyridin-2-yl)-imidazo[1,2-a]pyridine

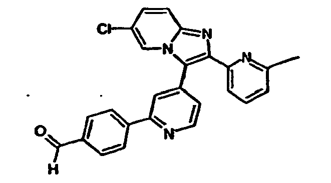

Intermediate 25 (1.15g, 2.88 mmol) and 4-formylphenyl boronic acid (563mg, 3.75 mmol) were reacted and treated as described for intermediate 30 to afford, after purification by chromatography on silica gel (CH₂Cl₂/MeOH 90:10), the title compound (1.15g, 93%); [APCI MS] m/z 425 (MH+).

### Intermediate 35: 7-Methyl-3-(2-(4-formyl-phenyl)-pyridin-4-yl)-2-(6-methyl-pyridin-2-yl)-imidazo[1,2-a]pyridine

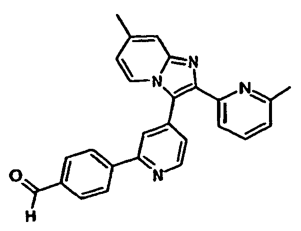

Intermediate 26 (1.43g, 3.78 mmol) and 4-formylbenzene boronic acid (738mg, 4.92 mmol) were coupled and treated as described for intermediate 30 to afford, after purification by flash chromatography on silica gel (CH₂Cl₂/MeOH 95:5), the title compound as an orange foam (270mg, 18%); [APCI MS] m/z 405 (MH+).

### Intermediate 36: 8-Methyl-3-(2-(4-formyl-phenyl)-pyridin-4-yl)-2-(6-methyl-pyridin-2-yl)-imidazo[1,2-a]pyridine

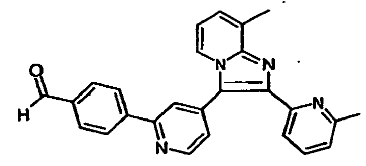

Intermediate 27 (1.5g, 3.97 mmol) and 4-formylphenyl boronic acid (0.773g, 5.16 mmol) were coupled and treated as described for intermediate 30 to afford the title compound as a cream powder (1.39g, 86%); ¹H NMR (300 MHz, CDCl₃) δ ppm:10.1 (s, 1H), 8.9 (d, 1H), 8.2 (d, 2H), 8.15 (s, 1H), 8.1 (d, 1H), 8 (d, 2H), 7.85 (d, 1H), 7.6 (m, 2H), 7.1 (m, 2H), 6.8 (t, 1H), 2.8 (s, 3H), 2.4 (s, 3H).

### Intermediate 37: 3-{2-[4-(Carboxy)-phenyl]-pyridin-4-yl}-2-(6-methyl-pyridin-2-yl)-imidazof 1 .2-alpyridine

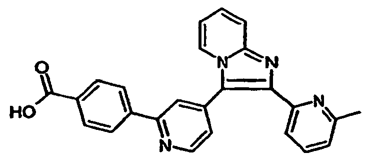

Intermediate 22 (2g, 5.49 mmol) and 4-(4,4,5,5-tetramethyl-[1,3,2]-dioxaborolan-2-yl)-benzoic acid (2.04g, 8.24 mmol) were coupled and treated as described for intermediate 30 to afford, after trituration with CH₂CI₂/diisopropyl ether, the title compound (1.4g, 62-76%); [APCI MS] m/z 407 (MH+).

### Intermediate 38: 3-(2-(4-Hydroxy-phenyl)-pyridin-4-yl)-2-(pyridin-2-yl)-imidazo[1.2-a]pyridine

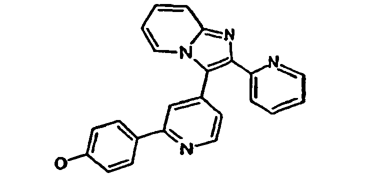

Intermediate 21 (1.85g, 5.27 mmol) and 4-(4,4,5,5-tetramethyl-[1,3,2]-dioxaborolan-2-yl)-phenol (1.5 g, 6.85 mmol) were coupled and treated as described for intermediate 30 to afford, after purification by flash chromatography on silica gel (CH₂Cl₂/MeOH, 98:2 then 95:5 then 93:7), the title compound as a solid (1.4 g, 73%); [APCl MS] m/z = 365 (MH+).

### Intermediate 39: 3-(2-(4-Hydroxy-phenyl)-pyridin-4-yl)-2-(6-methyl-pyridin-2-yl)-imidazo[1,2-a]pyridine

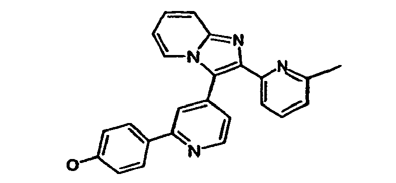

Intermediate 22 (1g, 2.74 mmol) and 4-(4,4,5,5-tetramethyt-[1,3,2]-dioxaborolan-2-yl)-phenol (786mg, 3.57 mmol) were coupled and treated as described for intermediate 30 to afford, after purification by chromatography on silica gel (CH₂Cl₂/MeOH 90:10), the title compound (470 mg, 45%); [APCI MS] m/z 379 MH⁺.

### Intermediate 40: 3-(2-(4-Hydroxy-phenyl)-pyridin-4-yl)-2-(3-chloro-phenyl)-imidazo[1,2-a]pyridine

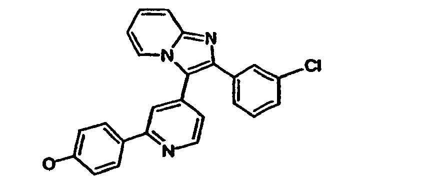

Intermediate 28 (3g, 7.83 mmol) and 4-(4,4,5,5-tetramethyl-[1,3,2]-dioxaborolan-2-yl)-phenol (2.24g, 10.2 mmol) were coupled and treated as described for intermediate 30 to afford, after chromatography on silica gel (CH₂Cl₂/MeOH 95:5), the title compound (1.6g, 51%); ¹H NMR (300 MHz, CDCl₃) δ ppm: 9.05 (s, 1H), 8.55 (d, 1H), 7.95 (d, 1H), 7.6 (m, 3H), 7.5 (m, 2H), 7.25 (m, 1H), 7.1 (m, 4H), 6.7 (m, 3H).

### Intermediate 41: 8-Methyl-3-(2-(4-hydroxy-phenyl)-pyridin-4-yl)-2-(6-methyl-pyridin-2-yl)-imidazo[1,2-a]pyridine

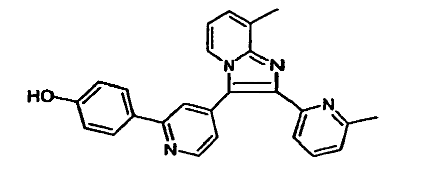

Intermediate 27 (2.06g, 5.45 mmol) and 4-(4,4,5,5-tetramethyl-[1,3.2]- dioxaborolan-2-yl)-phenol (1.56g, 7.08 mmol) were coupled and treated as described for intermediate 30 to afford the title compound as a brown powder (0.865g, 40%); [APCI MS] m/z 393 (MH⁺).

### Intermediate 42: 7-Methyl-3-(2-(4-hydroxy-phenyl)-pyridin-4-yl)-2-(6-methyl-pyridin-2-yl)-imidazo[1,2-a]pyridine

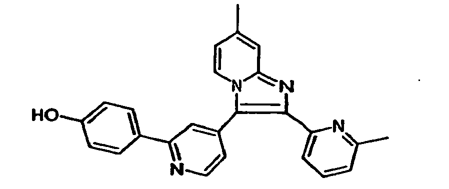

Intermediate 26 (1.17g, 3.1 mmol) and 4-(4,4,5,5-tetramethyl-[1,3,2]-dioxaborolan-2-yl)-phenol (0.55g, 4.02 mmol) were coupled and treated as described for intermediate 30 to afford the title compound (0.932g, 77%); [APCI MS] m/z 393 (MH⁺).

### Intermediate 43: 3-(2-(4-Bromo-phenyl)-pyridin-4-yl)-2-(pyridin-2-yl)-imidazo[1,2-a]pyridine

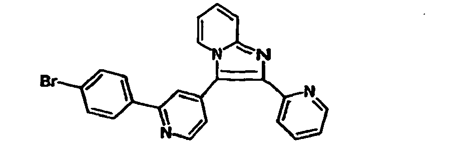

Intermediate 21 (3 g, 8.55 mmol) and 4-bromophenyl boronic acid (Aldrich, 2.23 g, 11.11 mmol) were coupled and treated as described for intermediate 30 to afford, after chromatography on silicagel (CH₂CI₂/MeOH 98:2 then 95: 5), the title compound as an oil (2.9 g, 79.5%); [APCI MS] m/z: 428.2 (MH+).

### Intermediate 44: 3-{2-[4-(2-Bromo-ethoxy)-phenyl]-pyridin-4-yl}-2-pyridin-2-yl-imidazo[1,2-a]pyridine

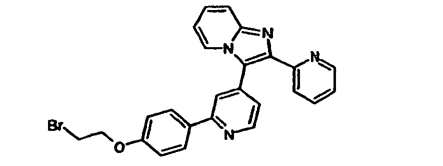

To a solution of intermediate 38 (0.38 g, 1.04 mmol) in acetone (20 ml) was added caesium carbonate (0.68 g, 2.08 mmol) and 1,2-dibromoethane (0.9 ml, 10.4 mmol) and the reaction was heated under reflux for 2 days. After cooling, the reaction was filtered and the solvent was removed in vacuo. Purification by chromatography on silica gel (CH₂Cl₂/MeOH, 90:10) gave the title compound (140 mg, 28%); ¹H NMR (CDCl₃, 300 MHz) δ ppm: 8.78 (d, 1H), 8.49 (d, 1 H), 8.14 (d, 1H), 7.93 (m, 4H), 7.72 (t, 2H), 7.34 (m, 2H), 7.17 (m, 1H), 7.00 (d, 2H), 6.83 (t, 1H), 4.33 (t, 2H), 3.65 (t, 3H).

### Intermediate 45: 3-{2-[4-(2-Bromo-ethoxy)-phenyl]-pyridin-4-yl}-2-(6-methyl-pyridin-2-yl)-imidazo[1,2-a]pyridine

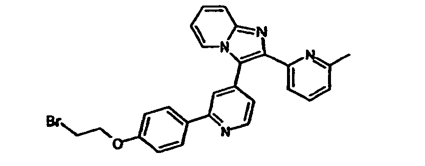

Intermediate 39 (0.46 g, 1.22 mmol) and 1,2-dibromoethane (2.08 ml, 24.32 mmol) were reacted as described for intermediate 44 to afford, after purification by chromatography on silica gel (CH₂Cl₂/MeOH, 95:5), the title compound (0.3g, 50%); ¹H NMR (CDCl₃, 300 MHz) δ ppm: 8.75 (d, 1H), 8.15 (d, 1H), 7.93 (m, 3H), 7.71 (t, 2H), 7.56 (t, 2H), 7.35 (d, 1H), 7.26 (m, 1H), 7.00 (m, 3H), 6.82 (t, 1H), 4.33 (t, 2H), 3.65 (t, 2H), 2.37 (s, 3H).

### Intermediate 46: 3-{2-[4-(2-Bromo-ethoxy)-phenyl]-pyridin-4-yl}-2-(6-chloro-phenyl)-imidazo[1,2-a]pyridine

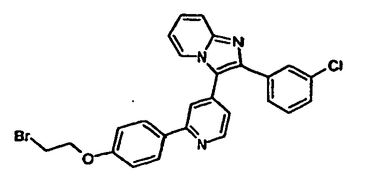

Intermediate 40 (1.6 g, 4 mmol) and 1,2-dibromoethane (4.37 ml, 50 mmol) were reacted and treated as described for intermediate 44 to afford, after purification by chromatography on silica gel (CH₂Cl₂/MeOH, 95:5), the title compound as an orange oil (2.98g, 100%); [APCI MS] m/z 505 (MH⁺).

### Intermediate 47: 7-Methyl-3-{2-[4-(2-bromo-ethoxy)-phenyl]-pyridin-4-yl}-2-(6-methyl-pyridin-2-yl)-imidazo[1,2-a]pyridine

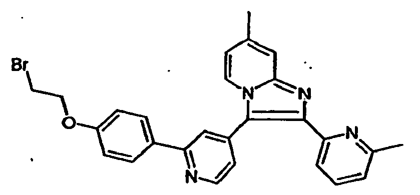

Intermediate 42 (3.72g, 9.49 mmol) and 1,2-dibromoethane (8.2 ml, 94.88 mmol) were reacted and treated as described for intermediate 44 to afford after purification by chromatography on silica gel (CH₂Cl₂/MeOH, 95/5) and trituration with pentane, the title compound as a yellow powder (1.28g, 27%); [APCI MS] m/z 500 (MH⁺).

### Intermediate 48: 8-Methyl-3-{2-[4-(2-bromo-ethoxy)-phenyl]-pyridin-4-yl}-2-(6-methyl-pyridin-2-yl)-imidazo[1,2-a]pyridine

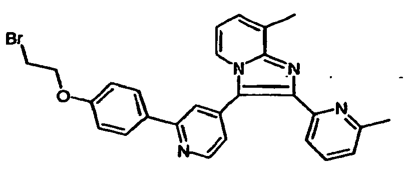

Intermediate 41 (0.865g, 2.2 mmol) and 1,2-dibromoethane (1.9 ml, 22.06 mmol) were reacted and treated as described for intermediate 44 to afford after purification by chromatography on silica gel (CH₂Cl₂/MeOH, 95:5), the title compound (0.389g, 35%); ¹H NMR (CDCl₃, 300 MHz) δ ppm: 8.75 (d, 1H), 8.05 (d, 1H), 7.95 (s, 1H), 7.9 (d, 2H), 7.7 (d, 1H), 7.55 (t, 1H), 7.35 (d, 1H), 7 (m, 2H), 6.9 (d, 2H), 6.75 (t, 1 H), 4.35 (t, 2H), 3.65 (t, 2H), 2.75 (s; 3H), 2.35 (s, 3H).

### Examples

### Example 1: 3-[2-(4-Methoxyphenyl)-pyridin-4-yl]-2-pyridin-2-yl-imidazo[1,2-a]pyridine

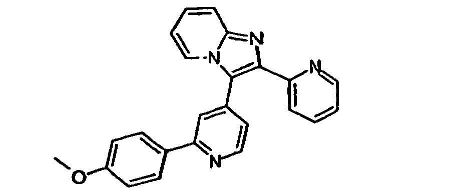

A solution of intermediate 21 (0.5g, 1.42 mmol) in toluene (10 ml) was treated with tetrakis(triphenylphosphine)palladium(O) (165mg, 10%mol) and stirred at room temperature for 30 min. Aqueous Na₂CO₃ (2M, 0.6 ml) was added to the reaction mixture followed by 4-methoxyphenyl boronic acid (0.282g, 1 .3eq, 1.85 mmol) and the mixture was heated under reflux overnight. The cooled mixture was poured into ice and extracted with toluene. The layers were separated and the organic layer was washed with water, dried over Na₂SO₄ and filtered. Evaporation of the filtrate in *vacuo* gave a crude oil which was purified by chromatography on silica gel (CH₂Cl₂/MeOH, 90:10) to give the title compound (68mg, 13%); m.p. 222°C; TOF MS ES⁺ exact mass calculated for C₂₄H₁₈N₄O : 379.1559 (MH+). Found 379.1540 (MH+).

### Example 2 : 3-[2-(4-methoxy-phenyl)-pyridin-4-yl]-2-(6-methyl-pyridin-2-yl)-imidazo[1,2-a]pyridine

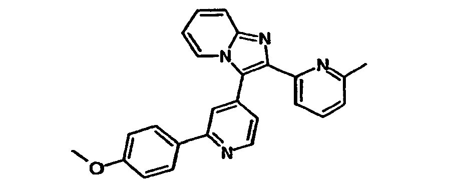

lntermediate 22 (300mg, 0.82mmol) and 4-methoxyphenyl boronic acid (162mg, 1.07mmol) were coupled and treated as described for example 1 to afford, after purification by chromatography on silica gel (CH₂Cl₂/MeOH, 95:5), the title product as a yellow powder (112mg, 35%); m.p. 174°C; [APCI MS] m/z 393 (MH⁺).

### Example 3:2-(6-methyl-pyridin-2-yl)-3-[2-(4-trifluoromethoxy-phenyl)-pyridin-4-yl]-imidazo[1,2-a]pyridine

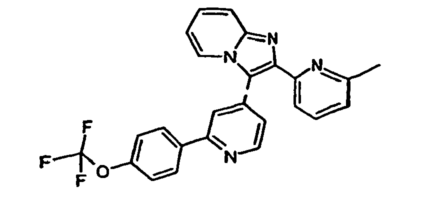

Intermediate 22 (300mg, 0.82mmol) and 4-trifluoromethoxybenzene boronic acid (220mg, 1.07mmol) were coupled and treated as described for example 1 to afford, after precipitation in pentane, the title product (137mg, 37%); m.p. 120°C; [APCl MS] m/z 447 (MH⁺).

### Example 4:3-[2-(4-Cyano-phenyl)-pyridin-4-yl]-2-(6-methyl-pyridin-2-yl)-imidazo[1,2-a]pyridine

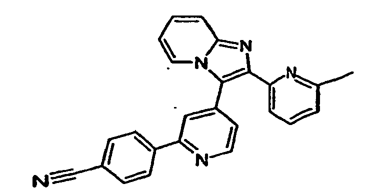

Intermediate 22 (300mg, 0.82mmol) and 4-cyanobenzene boronic acid (157mg, 1.07mmol) were coupled and treated as described for example 1 to afford, after recrystallisation from EtOAc, the title compound as a yellow powder (31mg, 10%); m.p. 214°C; [APCI MS] m/z 388 (MH⁺).

### Example 5: 3-[2-(4-Methanesulfonyl-phenyl)-pyridin-4-yl]-2-pyridin-2-yl-imidazo[1,2-a]pyridine

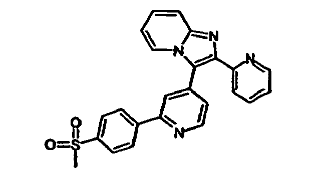

Intermediate 23 (1.5g, 4.3mmoo and 4-(rriethanesulfonyl)-phenyl boronic acid (1g, 5.1 mmol) were coupled and treated as described for example 1 to afford, after crystallisation in acetonitrile, the title compound as a pink powder (730mg. 40.33%); m.p. 242-244°C: [APCI MS] m/z 427 (MH+).

### Example 6: 3-[2-(4-Methanesulfonyl-phenyl)-pyridin-4-yl]-2-(6-methyl-pyridin-2-yl)-imidazo[1,2-a]pyridine

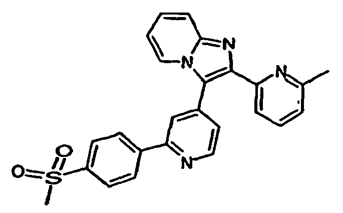

Intermediate 22 (300mg, 0.82mmol) and 4-(methanesulfonyl)-phenyl boronic acid (214mg, 1.06mmol) were coupled and treated as described for example 1 to afford, after purification by chromatography on silica gel (CH₂Cl₂/MeOH, 95:5), the title compound as a yellow foam (121 mg, 33%); [APCI MS] m/z 441 (MH+); ¹H NMR (300 MHz, CDCl₃) δ (ppm) 8.85 (d, 1 H); 8.2 (d, 1H); 8.14 (d, 1H); 8.09 (m, 3H); 7.82 (d, 1 H); 7.74 (d,1H); 7.58 (m, 2H); 7.53 (m, 1H); 7.44( dd, 1H); 7.3 (t, 1H); 7.05 (d,1H) ; 6.85 (t, 1H) ; 3.09 (s, 3H) ; 2.31 (s, 1H).

### Example 7: 8-Methyl-2-(6-methyl-pyridin-2-yl)-3-{2-[4-(methylsulfonyl)phenyl]-pyridin-4-yl}-imidazo[1,2-a]pyridine

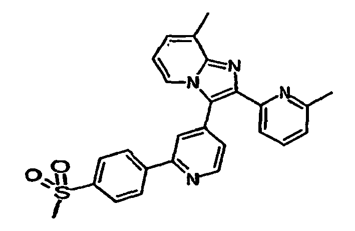

Intermediate 27 (0.5g, 1.3 mmol) and 4-(methylsulfonyl)phenyl boronic acid (0.344g, 1.72 mmol) were coupled and treated as described for example 1 to afford, after trituration with EtOAc, the title compound as a yellow gummy solid (250mg, 41 %); [APCI MS] m/z 455 (MH⁺); ¹H NMR (300 MHz, CDCl₃) δ (ppm) 8.85 (d, 1H), 8.2 (d, 2H), 8.05 (m, 3H), 7.85 (d, 1H), 7.5 (m, 3H), 7.1 (m, 2H), 6.8 (t, 1H), 3.1 (s, 3H), 2.75 (s, 3H), 2.35 (s, 3H).

### Example 8: 3-[2-(4-(Acetyl)phenyl)-pyridin-4-yl]-2-(pyridin-2-yl)-imidazo[1,2-a]pyridine

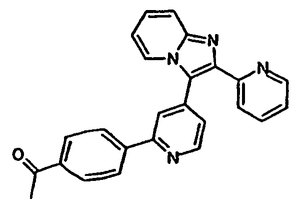

Intermediate 21 (2.1g, 5.98 mmol) and 4-(acetyl)phenyl boronic acid (1.27g, 7.77 mmol) were coupled and treated as described for example 1 to afford, after trituration with EtOAc, the title compound as a cream solid (1.9g, 81.4%); m.p. 214°C; [APCI MS] m/z 391.22 (MH⁺).

### Example 9: 3-[2-(4-(Methylcarbonylamino)phenyl)-pyridin-4-yl]-2-pyridin-2-yl-imidazo[1,2-a]pyridine

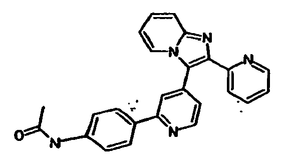

Intermediate 21 (0.3g, 0.85mmoQ and 4'-(4,4,5,5-tetramethyl-1,3,2-dioxoborolan-2-yl)-acetanilide (0.29 g, 1.11 mmol) were coupled and treated as described for example 1 to afford the title compound as a yellow powder (283mg, 82%); m.p. 133°C; [APCI MS] m/z 406 (MH+).

### Example 10: 3-[2-(4-(methylcarbonylamino)phenyl)-pyridin-4-yl]-2-(6-methyl-pyridin-2-yl)-imidazo[1,2-a]pyridine

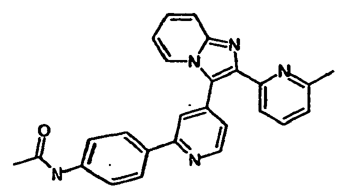

Intermediate 22 (3.76g, 10.32mmol) and 4'-(4,4,5,5-tetramethyl-1,3,2-dioxoborolan-2-yl)-acetanilide (3.5g, 13.42 mmol) were coupled and treated as described for example 1 to afford, after crystallisation from CH₂Cl₂, the title compound as a creme powder (2.34g, 54%); m.p. 257°C; TOF MS ES⁺ exact mass calculated for C₂₆H₂₁N₅O: 420.1824 (MH+). Found 420.1808 (MH⁺).

### Example 11 : 2-(3-chloro-phenyl)-3-[2-(4-(methanesulfonylamino)phenyl)-pyridin-4-yl]- imidazo[1,2-a]pyridine

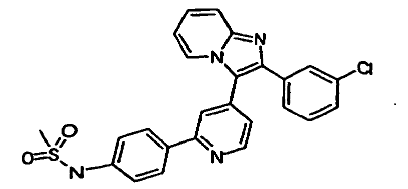

Intermediate 28 (300mg, 0.78mmol) and intermediate 12 (302mg, 1.02 mmol) were coupled and treated as described for example 1 to afford, after purification by flash chromatography on silica gel (CH₂Cl₂/MeOH, 95:5), the title compound as a yellow foam (93mg, 25%); m.p. 60°C (become gummy); TOF MS ES⁺ exact mass calculated for C₂₅H₁₉CIN₄O₂S : 475.0995 (MH+). Found: 475.0975 (MH+).

### Example 12 : 2-(6-methyl-pyridin-2-yl)-3-{2-[4-amino-phenyl]-pyridin-4-yl}-imidazo[1,2-a]pyridine

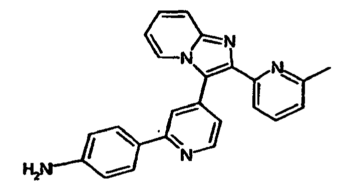

A mixture of example 10 (2.3g, 5.48 mmol) in MeOH (50 ml) and 1N HCI (50 ml) were stirred at room temperature for 18 hours. The mixture was then basified with 1 N NaOH and then extracted with CH₂Cl₂. The combined organic phases were dried over Na₂SO₄, filtered and concentrated under reduced pressure to give the title compound as a yellow solid (0.79g, 38%); [APCI MS] m/z: 378 (MH⁺); ¹H NMR (300 MHz, CDCl₃) δ ppm: 8.7 (d, 1H), 8.1 (d, 1 H), 7.85 (m, 3H), 7.7 (d, 1H), 7.6 (d, 1 H), 7.45 (t, 1H), 7.25 (m, 2H), 7 (d, 1 H), 6.8 (t, 1H). 6.7 (d, 2H), 3.85 (m, 2H), 2.4 (s, 3H).

### Example 13: 2-(6-Methyl-pyridin-2-yl)-3-{2-[4-trifluoromethylsulfonytamino)phenyl]-pyridin-4-yl}-imidazo[1,2-a]pyridine

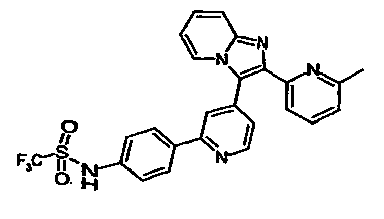

To a solution of example 12 (390mg, 1.03mmol) in CH₂Cl₂ (10ml) were added trifluoromethanesutfonic anhydride (0.2ml, 8.55mmol) and triethylamine (0.17 ml, 1.24 mmol) and the mixture was stirred at room temperature for 3 days. The mixture was poured into water and extracted with CH₂Cl₂. The organic phase was dried over Na₂SO₄, filtered and concentrated under reduced pressure. The residue was purified by chromatography on silica gel eluting with CH₂Cl₂ /MeOH (90:10) to give the title compound as a yellow powder (178mg, 33.8%); m.p. 135°C; TOF MS ES⁺ exact mass calculated for C₂₅H₁₈F₃N₅O₂S: 510.1212 (MH⁺). Found: 510.1229 (MH⁺).

### Example 14 : 3-[2-(4-(morpholin-4-yl)-phenyl)-pyridin-4-yl]-2-pyridin-2-yl-imidazo[1,2-a]pyridine

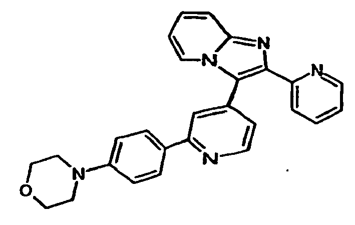

A mixture of intermediate 43 (400 mg, 0.93 mmol), morpholine (1.2 eq, 0.1 ml, 1.1 mmol), Pd₂(dba)₃ (0.05 eq, 43 mg, 0.05 mmol), BINAP (0.15 eq, 88 mg, 0.14 mmol) and potassium *tert*-butoxide (1.4 eq, 126 mg, 1.31 mmol) in toluene (50 ml) was heated under reflux for 2 hours. The reaction mixture was extracted between CH₂Cl₂ and water and combined organic extracts were dried over Na₂SO₄, filtered and concentrated under reduced pressure. The residue was purified by chromatography on silica gel eluting with CH₂Cl₂ /MeOH (98:2, 95:5 and then 93:7). The resulting oil was crystallised from CH₂Cl₂ /pentane to give the title compound as a yellow solid (140 mg, 35%); m.p. 145°C (become gummy); TOF MS ES⁺ exact mass calculated for C₂₇H₂₃N₅O: 434.1981 (MH+). Found 434.1993 (MH+).

### Example 15: 3-{2-[4-(4-Methylpiperazin-1-yl)-phenyl]-pyridin-4-yl}-2-pyridin-2-yl-imidazo[1,2-a]pyridine

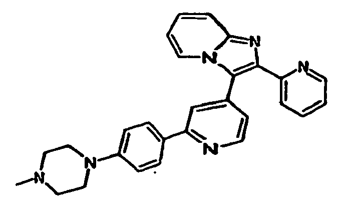

Intermediate 43 (400mg, 0.94mmol) and *N*-methyl-piperazine (0.125 ml, 1.2eq, 1.13 mmol) were coupled and treated as described for example 14 to afford, after crystallisation in CH₂Cl₂ /diisopropylether, the title compound (70mg, 17%); m.p. 150°C (become gummy); [APCI MS] m/z 447 (MH+).

### Example 16: 2-(6-Methyl-pyridin-2-yl)-3-[2-(4-(morpholin-4-ylmethyl)phenyl)-pyridin-4-yl]-imidazo[1,2-a]pyridine

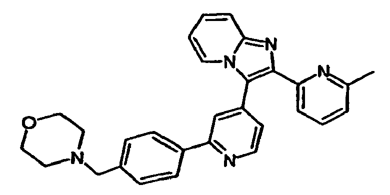

To a solution of intermediate 30 (310mg, 0.79mmol) and morpholine (1.5 eq, 0.1 ml, 1.2mmol) in dry dichloroethane (30ml) was added sodium triacetoxyborohydride (1.5eq, 253mg, 1.2 mmol) and the mixture stirred for 3 hours at room temperature. The mixture was basified with 1 N NaOH and the aqueous layer was extracted with CH₂Cl₂. The combined organic extracts were dried over Na₂SO₄, filtered and the filtrate was concentrated. The concentrate was recrystallised from ethyl acetate to give the title compound as a white powder (194mg, 53%); m.p. 156°C; [APCI MS] m/z 462.28 (MH+).

### Example 17: 3-[2-(4-(Morpholin-4-yl-methyl)-phenyl)-pyridin-4-yl]-2-pyridin-2-yl-imidazo[1,2-a]pyridine

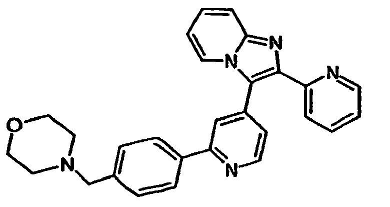

Intermediate 31 (1.1g, 2.9mmol) and morpholine (307µl. 3.5mmol) were coupled and treated as described for example 16 to afford, after purification by chromatography on silica gel (CH₂Cl₂ /MeOH, 90:10), the title compound as a powder (1.1g, 85%) m.p. 80°C (degradation); [APCI MS] m/z 448 (MH⁺).

### Example 18:2-(3-Chloro-4-fluoro-phenyl)-3-[2-(4-(morpholin-4-yl-methyl)phenyl)-pyridin-4-yl]-imidazo[1,2-a]pyridine

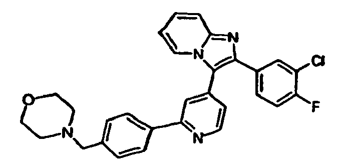

Intermediate 32 (0.35g, 0.82mmol) and morpholine (0.107m). 1.5 eq, 1.23 mmol) were coupled and treated as described for example 16 to afford, after crystallisation from EtOAc/iPr₂O, the title compound (45 mg, 11 %); m.p. 189°C; [APCI MS] m/z 499 (MH+).

### Example 19: 2-(3,4-Difluoro-phenyl)-3-[2-(4-(pyrrolidin-1-yl-methyl)phenyl)-pyridin-4-yl]-imidazo[1,2-a]pyridine

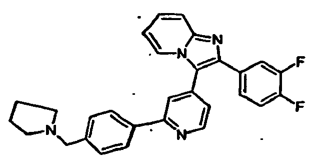

Intermediate 33 (0.30g, 0.73mmol) and pyrrolidine (0.09ml, 1.5 eq, 1.1 mmol) were coupled and treated as described for example 16 to afford, after crystallisation from CH₂Cl₂/pentane, the title compound as a yellow powder (51 mg, 45%); m.p. 155°C; TOF MS ES⁺ exact mass calculated for C₂₉H₂₄F₂N₄ : 467.2047 (MH+). Found 467.2063 (MH+).

### Example 20: 2-(3,4-Difluoro-phenyl)-3-[2-(4-(morpholin-1-yl-methyl)phenyl)-pyridin-4-yl]-imidazo-[1,2-a]pyridine

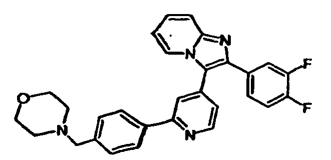

Intermediate 33 (0.30g, 0.73mmol) and morpholine (0.095mL, 1.1 mmol) were coupled and treated as described for example 16 to afford, after crystallisation from CH₂Cl₂ /pentane, the title compound as a white powder (135 mg, 38%); m.p. 205°C; TOF MS ES⁺ exact mass calculated for C₂₉H₂₄F₂N₄O: 483.1996 (MH+). Found 483.2030 (MH+).

### Example 21: 6-Chloro-2-(6-methyl-pyridin-2-yl)-3-[2-(4-(morpholin-4-yl-methyl)phenyl)-pyridin-4-yl]-imidazo[1,2-a]pyridine

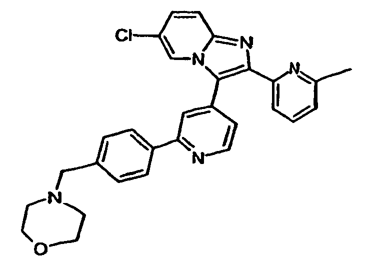

Intermediate 34 (0.40g, 0.94mmol) and morpholine (0.123 ml, 1.41 mmol) were coupled and treated as described for example 16 to afford, after crystallisation from diethyl ether, the title compound (129 mg, 28%); m.p. 157°C; [APCl MS] m/z 496 (MH⁺).

### Example 22: 7-Methyl-2-(6-methyl-pyridin-2-yl)-3-[2-(4-(morpholin-4-yl-methyl)phenyl)-pyridin-4-yl]-imidazo[1,2-a]pyridine

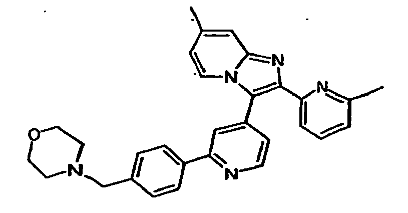

Intermediate 35 (270 mg, 0.66 mmol) and morpholine (0.09 ml, 1 mmol) were coupled and treated as described for example 16 to afford, after crystallisation from EtOAc, the title compound as an orange solid (68 mg, 22%); m.p. 188°C; TOF MS ES⁺ exact mass calculated for C₃₀H₂₉N₅O: 476.2450 (MH+). Found: 476.2445 (MH+).

### Example 23: 6-Chloro-2-(6-methyl-pyridin-2-yl)-3-[2-(4-(pyrrolidin-1-yl-methyl)phenyl)-pyridin-4-yl]-imidazo[1,2-a]pyridine

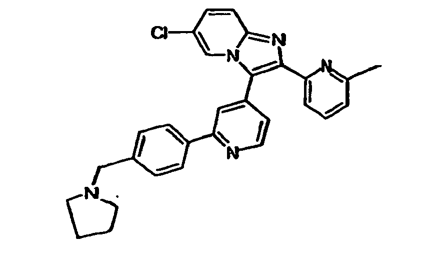

Intermediate 34 (0.30g, 0.7mmol) and pyrrolidine (0.09 ml, 1.06 mmol) were coupled and treated as described for example 16 to afford, after purification by chromatography on silica gel eluting with CH₂Cl₂/MeOH (90:10 then 80:20), the title compound as a white powder (122 mg, 36%); m.p. 134°C; TOF MS ES⁺ exact mass calculated for C₂₉H₂₆CIN₅: 480.1955 (MH⁺) found 480.1900 (MH+).

### Example 24: 8-Methyl-2-(6-methyl-pyridin-2-yl)-3-{2-[4-((morpholin-4-yl)methyl)phenyl]-pyridin-4-yl}-imidazo[1,2-a]pyridine

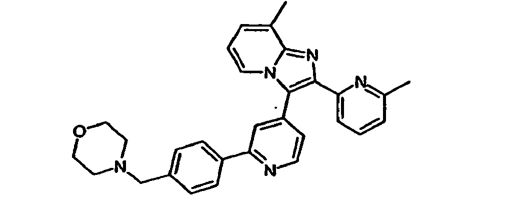

Intermediate 36 (400mg, 0.99 mmol) and morpholine (0.13ml, 1.49 mmol) were coupled and treated as described for example 16 to afford, after tritu ration with CH₂Cl₂/pentane, the title compound as a white solid (198mg, 42.13%); m.p. 122°C; [APCI MS] m/z 476 (MH⁺).

### Example 25: 8-Methyl-2-(6-methyl-pyridin-2-yl)-3-{2-[4-((pyrrolidin-1-yl)methyl)phenyl]-pyridin-4-yl}-imidazo[1,2-a]pyridine, hydrochloride

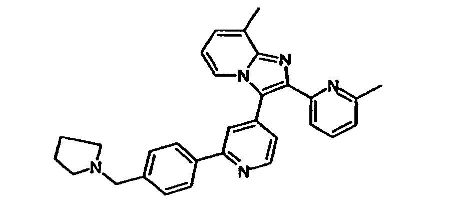

To a solution of intermediate 36 (400mg, 0.99mmol) in CH₂Cl₂(20ml) were added pyrrolidine (0.13ml, 1.49mmol) and sodium triacetoxyborohydride (315mg, 1.49mmol) and the mixture was stirred at room temperature for 24 hours. The mixture was poured into water and extracted with CH₂Cl₂. The phases were separated and the combined organic extracts were dried over Na₂SO₄, filtered and the filtrate was concentrated under reduced pressure. The residue was dissolved in CH₂Cl₂ and a solution of hydrochloric acid in diethyl ether (1N, 1.3 ml, 1.3mmol) was added. The resulting precipitate was filtered, washed with diisopropyl ether and dried to give the title compound as a yellow solid (322mg, 71%); m.p. 197°C: ¹H NMR (300 MHz, DMSO- d₆) δ ppm: 8.95 (d, 1H), 8.45 (m, 2H), 8.2 (d, 2H), 7.95 (t, 1H), 7.8 (d, 2H), 7.75 (m, 3H), 7.5 (d, 1H), 7.25 (t, 1H), 4.4 (m, 2H), 3.3 (m, 2H), 3.05 (m, 2H), 2.95 (s, 3H), 2.5 (s, 3H), 2.05 (m, 2H), 1.9 (m, 2H).

### Example 26: 3-[2-(4-((Tetrahydropyran-4-yl)aminocarbonyl)phenyl)-pridin-4-yl]-2-pyridin-2-yl-imidazo[1,2-a]pyridine

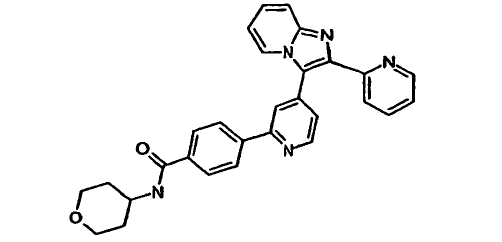

Intermediate 21 (0.224g, 0.638 mmol) and intermediate 10 (0.206g, 0.83 mmol) were coupled and treated as described for example 1 to afford, after purification by chromatography on silica gel (CH₂Cl₂/MeOH 90:10), the title compound as a yellow powder (0.057g, 19%); m.p. 179°C; [APCI MS] m/z 476 (MH+).

### Example 27: 3-[2-(4-((tetrahydropyran-4-yl)aminocarbonyl)phenyl)-pyridin-4-yl]-2-(6-methyl-pyridin-2-yl)-imidazo[1,2-a]pyridine

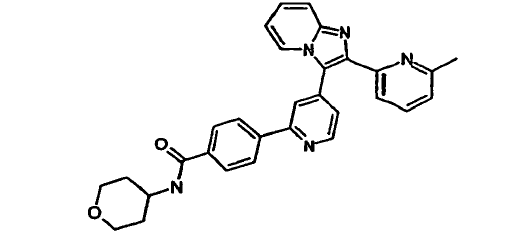

Intermediate 22 (300mg, 0.82 mmol) and intermediate 10 (266mg, 1.07 mmol) were coupled and treated as described for example 1 to afford, after purification by chromatography on silica gel (CH₂CI₂/MeOH, 90:10), the title compound as a yellow powder (37mg, 9%); m.p. 128°C; [APCI MS] m/z 490 (MH+).

### Example-28: 2-(3-Chloro-phenyl)-3-[2-(4-((tetrahydropyran-4-yl)aminocarbonyl)phenyl)-pyridin-4-yl]- imidazo[1,2-a]pyridine

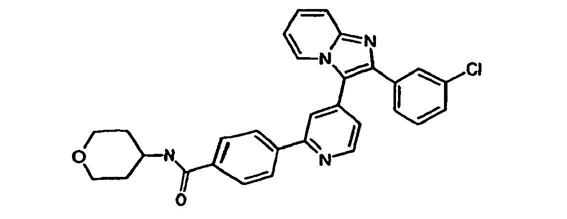

Intermediate 28 (300mg, 0.78mmol) and intermediate 10 (253mg, 1.02 mmol) were coupled and treated as described for example 1 to afford, after purification by preparative plate chromatography on silica gel (CH₂Cl₂/MeOH 90:10), the title compound as a yellow powder (51mg, 13%); m.p. 234°C; ¹H NMR (300 MHz, CDCl₃) δ ppm :7.85 (d, 2H). 7.75 (d, 2H), 7.65 to 7.55 (m, 4H), 7.45 to 7.35 (m, 6H), 7.2 (m, 1H), 6 (d, 1 H), 4.2 (m, 1H), 4 (m, 2H), 3.5 (m, 2H), 2 (m, 2H), 1.6 (m, 2H).

### Example 29: 2-(6-Methyl-pyridin-2-yl)-3-{2-[4-((morpholin-4-yl)carbonyl)phenyl]-pyridin-4-yl}-imidazo[1,2-a]pyridine

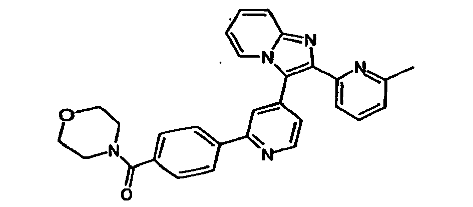

To a solution of intermediate 37 (500mg, 1.23mmol) in DMF (30ml) were added morpholine (0.13ml, 1.48mmol), HOBT (200mg, 1.48mmol), EDCI (283mg, 1.48mmol) and triethylamine (0.2ml; 1 .48mmol) and the mixture was stirred at room temperature overnight. The reaction mixture was extracted between CH₂Cl₂ and 1 N sodium hydroxide solution. The phases were separated and the organic phase was washed with water, dried over Na₂SO₄, filtered, and the filtrate was concentrated under reduced pressure. The residue was purified by chromatography on silica gel, eluting with CH₂Cl₂/MeOH (95 :5) to give (after trituration with diisopropyl ether) the title compound as a pale yellow solid (147mg, 25.13%); m.p. 110°C; [APCl MS] m/z 476.33 (MH+).

### Example 30: 2-(6-Methyl-pyridin-2-yl)-3-{2-[4-((3-methoxypropylamino)carbonyl)phenyl]-pyridin-4-yl}-imidazo[1,2-a]pyridine

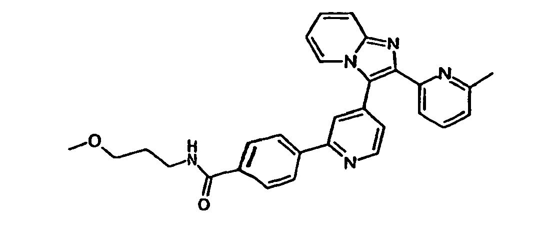

Intermediate 37 (400mg, 0.98mmol) and 3-methoxypropylamine (0.11ml, 1. 18mmol) were coupled and treated as described for example 29 to afford, after trituration with CH₂Cl₂/pentane, the title compound as a pale yellow solid (210mg, 44.69%); m.p. 165°C; [APCI MS] m/z 478.36 (MH⁺).

### Example 31: 8-Methyl-2-(6-methyl-pyridin-2-yl)-3-{2-[4-((morpholin-4-yl)carbonyl)phenyl]-pyridin-4-yl}-imidazo[1,2-a]pyridine

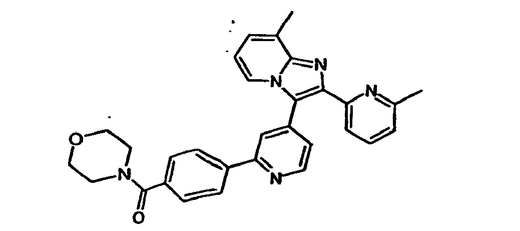

Intermediate 27 (500mg, 1.32mmol) and intermediate 12 (545mg, 1.72mmol) were coupled and treated as described for example 1 to afford the title compound as a yellow oil (543mg, 84%); [APCI MS] m/z 490 (MH+).

### Example 32: 8-Methyl-2-(6-methyl-pyridin-2-yl)-3-{2-[4-((1-ethyl-piperazin-4-yl)carbonyl)phenyl]-pyridin-4-yl}-imidazo[1,2-a]pyridine

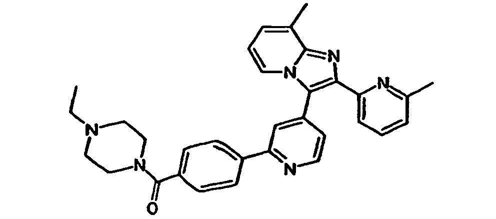

Intermediate 27 (500mg, 1.32mmol) and intermediate 13 (591mg, 1.72mmol) were coupled and treated as described for example 1 to afford the title compound (316mg, 46%); m.p. 212°C; TOF MS ES⁺ exact mass calculated for C₃₂H₃₂N₆O: 517.2715 (MH⁺). Found: 517.2751(MH⁺).

### Example 33: 2-(6-Fluoro-pyridin-2-yl)-3-{2-[4-((morpholiny-4-yl)carbonyl)phenyl]-pyridin-4-yl}-imidazo[1,2-a]pyridine

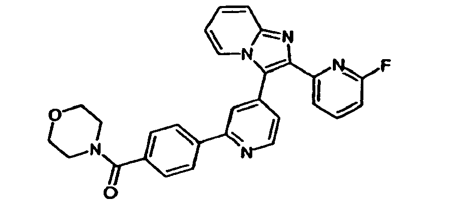

Intermediate 29 (200mg, 0.54mmol) and intermediate 12 (224mg, 0.7mmol) were coupled and treated as described for example 1 to afford the title compound as a white solid (30mg, 12%); m.p. 191°C; TOF MS ES⁺ exact mass calculated for C₂₈H₂₂N₅O₂F: 480.1836 (MH⁺). Found: 480.1756 (MH⁺).

### Example 34: 2-(Pyridin-2-yl)-3-{2-[4-(2-(pyrrolidin-1-yl)ethoxy)phenyl]-pyridin-4-yl}-imidazo[1,2-a]pyridine

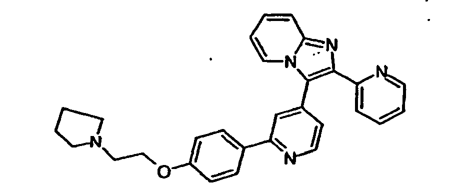

A solution of intermediate 44 (140 mg, 0.3mmol) and pyrrolidine (0.75ml, 9 mmol) in EtOH (5 ml) was heated under reflux for 6 days. After cooling water was added and the product was extracted with CH₂Cl₂. The organic phase was dried over Na₂SO₄, filtered, and the filtrate was evaporated under reduced pressure. The residue was purified by chromatography on silica gel eluting with CH₂Cl₂/MeOH/TEA (80:20:1%) to give the title compound (13 mg, 10%); [APCI MS] m/z 462 (MH+); ¹H NMR (300 MHz, CDCl₃) δ ppm: 8.75 (d, 1H), 8.5 (d, 1 H), 8.15 (d, 1H), 7.9 (m, 3H), 7.85 (s, 1H), 7.7 (m, 2H), 7.3 (m, 2H), 7.2 (m, 1H), 7 (d, 2H), 6.85 (t, 1H), 4.2 (t, 2H), 3 (t, 2H), 2.75 (m, 4H), 1.85 (m, 4H).

### Example 35: 2-(6-Methyl-pyridin-2-yl)-3-{2-[4-(2-(dimethylamino)ethoxy)phenyl]-pyridin-4-yl}-imidazo[1,2-a]pyridine

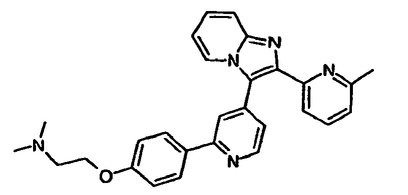

A solution of intermediate 45 (300 mg, 6.2 mmol) and dimethylamine (solution 40% in water, 2ml) in THF (2ml) was stirred at room temperature for 18 hours. After cooling water was added and the product was extracted with CH₂Cl₂. The organic phase was dried over Na₂SO₄, filtered and the solvent was removed under reduced pressure to give the title compound as a orange gum (135 mg, 48%); [APCI MS] m/z 450 (MH+); ¹H NMR (300 MHz, CDCl₃) δ ppm: 8.8 (d, 1H), 8.2 (d, 1H), 7.95 (m, 3H), 7.75 (m, 2H), 7.6 (t, 1H), 7.4 (d, 1H), 7.3 (m, 1H), 7.05 (m, 3H), 6.9 (t, 1 H), 4.25 (t, 2H), 3 (t, 2H), 2.55 (s, 6H), 2.4 (s, 3H).

### Example 36: 2-(3-Chloro-phenyl)-3-{2-[4-(2-(dimethylamino)ethoxy)phenyl]-pyridin-4-yl}-imidazo[1,2-a]pyridine

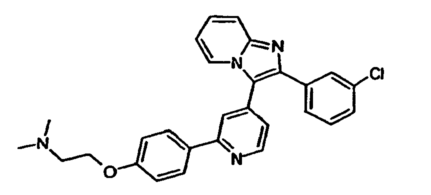

Intermediate 46 (300 mg, 0.6 mmol) and dimethylamine (solution 40% in water, 2ml) were coupled and treated as described for example 35 to afford, after purification by chromatography on silica gel eluting with CH₂Cl₂/MeOH (90:10 then 80:20), the title compound as a yellow gum (98 mg, 35%); TOF MS ES⁺ exact mass calculated for C₂₈H₂₅ClN₄O : 469.1795 (MH⁺). Found: 469.1723 (MH+); ¹H NMR (300 MHz, CDCl₃) δ ppm: 8.8 (d, 1H), 8.1 (d, 1 H), 7.9 (d, 2H), 7.8 (s, 1 H), 7.7 (m, 2H), 7.45 (d, 1H), 7.25 (m, 3H), 7.2 (m, 1H), 7 (d, 2H), 6.85 (t, 1H), 4.35 (t, 2H), 3.25 (t, 2H), 2.7 (s, 6H).

### Example 37: 2-(3-Chloro-phenyl)-3-{2-[4-(2-(pyrrolidin-1-yl)ethoxy)phenyl]-pyridin-4-yl}-imidazo[1,2-a]pyridine

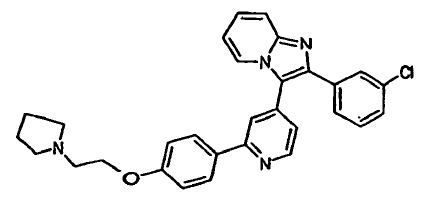

Intermediate 46 (300mg, 0.59mmol) and pyrrolidine (0.5ml, 5.96mmol) were coupled and treated as described for example 34 to afford, after trituration with pentane, the title compound as a pale yellow solid (80mg, 27.1%); m.p. 298°C; TOF MS ES⁺ exact mass calculated for C₃₀H₂₇ClN₄O: 495.1952 (MH+). Found: 495. 1957(MH+).

### Example 38: 7-Methyl-2-(6-methyl-pyridin-2-yl)-3-{2-[4-(2-(pyrrolidin-1-yl)ethoxy)phenyl]-pyridin-4-yl}-imidazo[1,2-a]pyridine

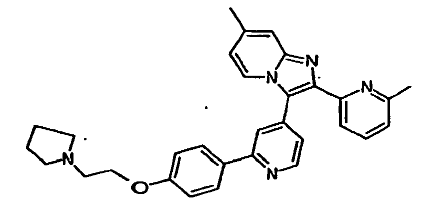

Intermediate 47 (426mg, 0.85mmol) and pyrrolidine (0.71ml, 8.55mmol) were coupled and treated as described for example 34 to afford, after trituration with pentane, the title compound as a pale yellow solid (102mg, 24.4%); m.p. 163°C; TOF MS ES⁺ exact mass calculated for C₃₁H₃₁N₅O: 490.2607 (MH⁺). Found: 490.2600 (MH⁺).

### Example 39: 7-Methyl-2-(6-methyl-pyridin-2-yl)-3-{2-[4-(2-(morpholin-4-yl)ethoxy)phenyl]-pyridin-4-yl}-imidazo[1,2-a]pyridine

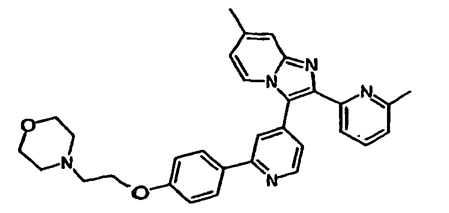

Intermediate 47 (426mg, 0.85mmol) and morpholine (0.74ml, 8.55mmol) were coupled and treated as described for example 34 to afford the title compound as an orange foam (357mg, 82.64%); TOF MS ES⁺ exact mass calculated for C₃₁H₃₁N₅O₂ 506.2556 (MH⁺). Found: 506.2534 (MH⁺): ¹H NMR (300 MHz, CDCl₃) δ ppm: 8.8 (d, 1H), 8.05 (d, 1H), 7.9 (m, 3H), 7.7 (d, 1H), 7.6 (t, 1H), 7.5 (s, 1 H), 7.35 (d, 1 H), 7 (m, 3H), 6.7 (d, 1H), 4.2 (t, 2H), 3.8 (m, 4H), 2.85 (t, 2H), 2.6 (m, 4H), 2.45 (s, 3H), 2.4 (s, 3H).

### Example 40: 8-Methyl-2-(6-methyl-pyridin-2-yl)-3-{2-[4-(2-(pyrrolidin-1-yl)ethoxy))phenyl]-pyridin-4-yl}-imidazo[1,2-a]pyridine

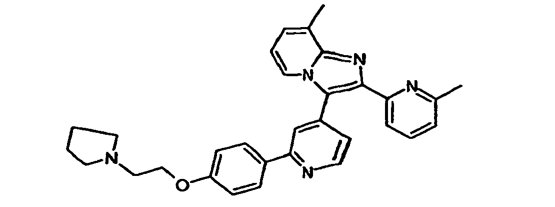

Intermediate 48 (389mg, 0.78mmol) and pyrrolidine (0.65ml, 7.8mmol) were coupled and treated as described for example 34 to afford the title compound as a gummy solid (160mg, 41.9%); [APCl MS] m/z 490 (MH+); ¹H NMR (300 MHz, CDCl₃) δ ppm: 8.75 (d, 1H), 8.05 (d, 1H), 7.9 (m, 3H), 7.7 (d, 1H), 7.55 (t, 1H), 7.3 (d, 1H), 7.05 (m, 2H), 6.95 (d, 2H), 6.7 (t, 1H), 4.25 (t, 2H), 3.05 (t, 2H), 2.9 (m, 4H), 2.7 (s, 3H), 2.4 (s, 3H), 1.95 (m, 4H).

### Example 41: 7-Methyl-2-(6-methyl-pyridin-2-yl)-3-{2-[4-((1-methyl-imidazol-4-yl)methyloxy)phenyl]-pyridin-4-yl}-imidazo[1,2-a]pyridine

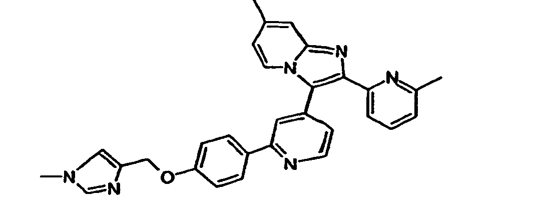

To a solution of intermediate 42 (0.4g, 1.02mmol) in DMF (20ml) was added portionwise sodium hydride (60% in mineral oil, 101mg, 2.55mmol) and the mixture was stirred at room temperature for 20 minutes. Intermediate 7 (173mg, 1.32mmol) was then added and the mixture was heated at 60°C for 3 days. The reaction mixture was poured into water and extracted with CH₂Cl₂. The combined organic extracts were separated, dried over Na₂SO₄, filtered and the filtrate was concentrated under reduced pressure. The residue was purified by chromatography on silica gel eluting with CH₂Cl₂/MeOH (90:10), to give (after trituration with diisopropyl ether), the title compound as a yellow solid (130mg, 26%); m.p. 217°C; TOF MS ES⁺ exact mass calculated for C₃₀H₂₆N₆O: 487.2246 (MH⁺). Found: 487.2247 (MH⁺).

### Example 42: 2-(6-Methyl-pyridin-2-yl)-3-{2-[4-((1-methyl-imidazol-4-yl)methytoxy)phenyl]-pyridin-4-yl}-imidazo[1,2-a]pyridine

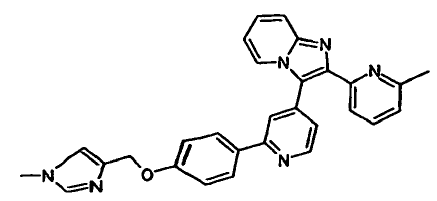

Intermediate 39 (400mg, 1.06mmol) and intermediate 7 (212mg, 1.27mmol) were coupled and treated as described for example 41 to afford, after trituration with diisopropyl ether, the title compound as a white solid (200mg, 40%); m.p. 120°C; [APCl MS] m/z 473 (MH+).

### Example 43: 7-Methyl-2-(6-methyl-pyridin-2-yl)-3-{2-[4(aminocarbonylmethyloxy)phenyl]-pyridin-4-yl}-imidazo[1,2-a]pyridine

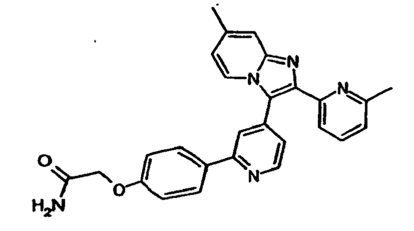

To a solution of intermediate 42 (500mg, 1.27mmol) in acetone (25ml) were added caesium carbonate (623mg, 1.91mmol) and bromoacetamide (264mg, 1.91mmol) and the mixture was heated under reflux for 48 hours. On cooling, the reaction mixture was extracted between water and CH₂Cl₂. The layers were separated and the combined organic layers were dried over Na₂SO₄, filtered and the filtrate was concentrated under reduced pressure- The residue was purified by chromatography on silica gel eluting with CH₂Cl₂/MeOH (95:5), to give (after trituration with pentane/ethyl acetate), the title compound (133mg, 23%); m.p. 213°C; [APCI MS] m/z 450 MH⁺.

### Example 44: 8-Methyl-2-(6-methyl-pyridin-2-yl)-3-(2-[4-(aminocarbonylmethyloxyl)phenyl]-pyridin-4-yl)-imidazo[1,2-a]pyridine

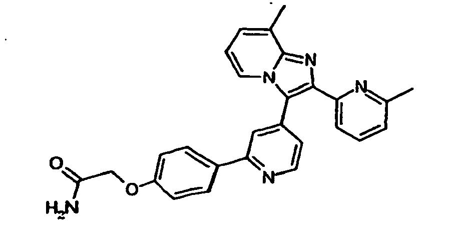

Intermediate 41 (0.5g, 1.27mmol) and bromoacetamide (0.264g. 1.91mmol) were coupled and treated as described for example 43 to afford, after trituration with diisopropyl ether, the title compound (0.08g, 14%); m.p. 183°C; [APCI MS] m/z 450 (MH+).

### Example 45: 2-(6-Methyl-pyridin-2-yl)-3-{2-[4-(morpholin-4-yl)phenyl]-pyridin-4-yl}-imidazo[1,2-a]pyridine

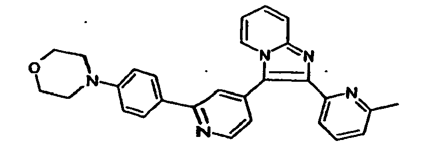

To a solution of intermediate 20 (3g, 8.04mmol) in CH₂Cl₂ (80 ml) was added bromine-polymer supported (5.03g, 8.04 mmol) and the suspension was stirred at room temperature for 3 hours. The suspension was filtered, washing the resin with ethanol. 2-Aminopyridine (1.51g , 16.08 mmol) was added to the combined filtrate and washings and the mixture was heated at reflux for 18 hours. On cooling, the residue was extracted between water and CH₂Cl₂. The combined organic phases were dried over Na₂SO₄, filtered and the filtrate was concentrated under reduced pressure. The concentrate was purified by chromatography on silica gel (eluti ng with CH₂Cl₂/MeOH, 98:2 then 95:5) to give (after trituration with diisopropyl ether, the title compound (1.2g; 33.38%); m.p. 190°C; TOF MS ES⁺ exact mass calculated for C₂₈H₂₅N₅O: 448.2137(MH⁺). Found: 448.2081 (MH⁺).

### Example 46 : 7-Methyl-2-(6-methyl-pyridin-2-yl)-pyridin-2-yl)-3-{2-[4-(morpholin-4-yl)phenyl]-pyridin-4-yl}-imidazo[1,2-a]pyridine

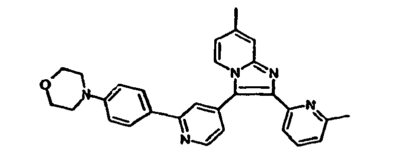

Intermediate 20 (1.27g, 3.4 mmol) was reacted as described for example 45, to afford after trituration with diisopropyl ether, the title compound (0.6g, 38.22%); m.p. 208°C; TOF MS ES⁺ exact mass calculated for C₂₉H₂₇N₅O: 462.2294(MH⁺). Found 462.2263 (MH⁺).

### Biology

The biological activity of the compounds of the invention may be assessed using the following assays:

### Assay 1 (Cellular transcriptional assay)

The potential for compounds of the invention to inhibit TGF- β signalling may be demonstrated, for example, using the following *in vitro* assay.

The assay was performed in HepG2 cells stably transfected with the PAI-1 promoter (known to be a strong TGF-β responsive promoter) linked to a luciferase (firefly) reporter gene. The compounds were selected on their ability to inhibit luciferase activity in cells exposed to TGF-β. In addition, cells were transfected with a second luciferase (Renilla) gene which was not driven by a TGF-β responsive promoter and was used as a toxicity control.

96 well microplates were seeded, using a muhidrop apparatus, with the stably transfected cell line at a concentration of 35000 cells per well in 200 µl of serum-containing medium. These plates were placed in a cell incubator.

18 to 24 hours later (Day 2), cell-incubation procedure was launched. Cells were incubated with TGF-β and a candidate compound at concentrations in the range 50 nM to 10 µM (final concentration of DMSO 1%). The final concentration of TGF-β (rhTGFβ-1) used in the test was 1 ng/mL Cells were incubated with a candidate compound 15-30 mins prior to the addition of TGF-β. The final volume of the test reaction was 150 µl. Each well contained only one candidate compound and its effect on the PAI-1 promoter was monitored.

Columns 11 and 12 were employed as controls. Column 11 contained 8 wells in which the cells were incubated in the presence of TGF-β, *without* a candidate compound. Column 11 was used to determine the 'reference TGF-β induced firefly luciferase value' against which values measured in the test wells (to quantify inhibitory activity) were compared. In wells A12 to D12, cells were grown in medium without TGF-β. The firefly luciferase values obtained from these positions are representative of the 'basal firefly luciferase activity'. In wells E12 to H12, cells were incubated in the presence of TGF-β and 500 µM CPO (Cyclopentenone, Sigma), a cell toxic compound. The toxicity was revealed by decreased firefly and renilla luciferase activities (around 50 % of those obtained in column 11).

12 to 18 hours later (day 3), the luciferase quantification procedure was launched. The following reactions were performed using reagents obtained from a Dual Luciferase Assay Kit (Promega). Cells were washed and lysed with the addition of 10 µl of passive lysis buffer (Promega). Following agitation (15 to 30 mins), luciferase activities of the plates were read in a dual-injector luminometer (BMG lumistar). For this purpose, 50 µl of luciferase assay reagent and 50 µl of 'Stop & Glo' buffer were injected sequentially to quantify the activities of both luciferases. Data obtained from the measurements were processed and analysed using suitable software. The mean Luciferase activity value obtained in wells A11 to H11 (Column 11, TGF-βonly) was considered to represent 100% and values obtained in wells A12 to D12 (cells in inedium alone) gave a basal level (0%). For each of the compounds tested, a concentration response curve was constructed from which an IC₅₀ value was determined graphically.

### Assay 2 (Alk5 Fluorescence Polarization Assay)

Kinase inhibitor compounds conjugated to fluorophores, can be used as fluorescent ligands to monitor ATP competitive binding of other compounds to a given kinase. The increase in depolarization of plane polarized light, caused by release of the bound ligand into solution, is measured as a polarization/anisotropy value. This protocol details the use of a rhodamine green-labelled ligand for assays using recombinant GST-ALK5 (residues 198-503).

Assay buffer components: 62.5 mM Hepes pH 7.5 (Sigma H-4034), 1 mM DTT (Sigma D-0632), 12.5 mM MgCl₂ (Sigma M-9272),1.25 mM CHAPS (Sigma C-3023).

Protocol: Solid compound stocks were dissolved in 100% DMSO to a concentration of 1 mM and transferred into column 1, rows A-H of a 96-well, U bottom, polypropylene plate (Costar #3365) to make a compound plate. The compounds were serially diluted (3-fold in 100% DMSO) across the plate to column 11 to yield 11 concentrations for each test compound. Column 12 contained only DMSO. A Rapidplate™-96 was used to transfer 1 µl of sample from each well into a 96-well, black, U-bottom, non-treated plate (Costar #3792) to create an assay plate.

ALK5 was added to assay buffer containing the above components and 1 nM of the rhodamine green-labelled ligand so that the final ALK5 concentration was 10 nM based on active site titration of the enzyme. The enzyme/ligand reagent (39 µl) was added to each well of the previously prepared assay plates. A control compound (1 µl) was added to column 12, rows E-H for the low control values. The plates were read immediately on a LJL Acquest fluorescence reader (Molecular Devices, serial number AQ1048) with excitation, emission, and dichroic filters of 485nm, 530 nm, and 505 nm, respectively. The fluorescence polarization for each well was calculated by the Acquest reader and then imported into curve fitting software for construction of concentration response curves. The normalized response was determined relative to the high controls (1 µl DMSO in column 12, rows A-D) and the low controls (1 µl of control compound in column 12, rows E-H). An IC₅₀ value was then calculated for each compound.

Using the above assays all Examples of the invention show ALK5 receptor modulator activity (having IC₅₀ values in the range of 1 to 200nM) and TGF-β cellular activity (having IC₅₀ values in the range of 0.001 to 10µM).

3-[2-(4-Methanesulfonyl-phenyl)-pyridin-4-yl]-2-(6-methyl-pyridin-2-yl)- imidazo[1,2-a]pyridine (Example 6) showed an ALK5 receptor modulator activity of 11 nM and TGF-β cellular activity of 125 nM.

7-Methyl-2-(6-methyl-pyridin-2-yl)-3-{2-[4-(2-(pyrrolidin-1-yl)ethoxy)phenyl]-pyridin-4-yl}-imidazo[1,2-a]pyridine (Example 38) showed an ALK5 receptor modulator activity of 15 nM and TGF-β cellular activity of 127 nM.

## Claims

1. A compound of formula (I), a pharmaceutically acceptable salt, solvate or derivative thereof: 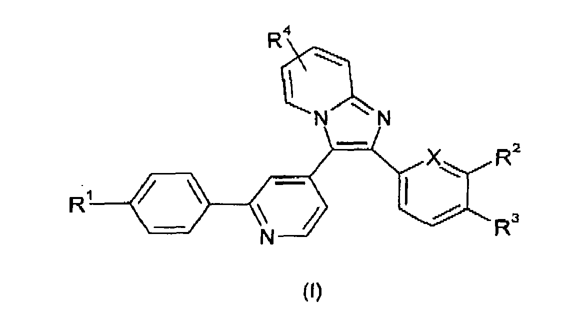 wherein
X is N or CH;
R¹ is selected from hydrogen, C₁₋₆alkyl. C₁₋₆alkenyl, C₁₋₆alkoxy, halo, cyano, perfluoro C₁₋₆alkyl, perfluoroC₁₋₆alkoxy, -NR⁵R⁶, -(CH₂)ₙNR⁵R⁶, -O(CH₂)ₙOR⁷, -O(CH₂)ₙ-Het, -O(CH₂)ₙNR⁵R⁶. -CONR⁵R⁶, -C(O)R⁷, -CO(CH₂)ₙNR⁵R⁶, -SO₂R⁷, -SO₂NR⁵R⁶, -NR⁵SO₂R⁷, -NR⁵COR⁷ and -O(CH₂)ₙCONR⁵R⁶;
R² is hydrogen, C₁₋₆alkyl, halo, cyano or perfluoroC₁₋₆alkyl;
R³ is hydrogen or halo;
R⁴ is hydrogen, halo, C₁₋₆alkyl or -NR⁵R⁶;
R⁵ and R⁶ are independently selected from hydrogen, C₁₋₆alkyl, perfluoroC₁₋₆ alkyl, Het or C₁₋₄alkoxyC₁₋₄alkyl; or R⁵ and R⁶ together with the nitrogen atom to which they are attached form a 3, 4, 5, 6 or 7-membered saturated or unsaturated ring which may contain one or more heteroatoms selected from N, S or O, and wherein the ring may be further substituted by one or more substituents selected from halo (such as fluoro, chloro, bromo), cyano, -CF₃, hydroxy, -OCF₃, C₁₋₆alkyl and C₁₋₆alkoxy;
R⁷ is hydrogen or C₁₋₆alkyl;
Het is a 5 or 6-membered C-linked heterocyclyl group which may be saturated, unsaturated or aromatic, which may contain one or more heteroatoms selected from N, S or O and which may be substituted by C₁₋₆alkyl; and
n is 1-4.

2. A compound according to claim 1 wherein X is N.

3. A compound according to any preceding claim wherein R¹ is -NR⁵R⁶, -(CH₂)ₙNR⁵R⁶, -O(CH₂)ₙNR⁵R⁶, -O(CH₂)ₙ-Het, -CONR⁵R⁶, -CO(CH₂)ₙNR⁵R⁶ or - S0₂R⁷.

4. A compound according to any preceding claim wherein R² is hydrogen, C₁₋₆alkyl, chloro or fluoro.

5. A compound according to any preceding claim wherein R³ is hydrogen or fluoro.

6. A compound according to any preceding claim wherein when X is N, R² is methyl.

7. A compound according to any preceding claim wherein when X is N and R² is methyl, R³ is hydrogen.

8. A compound according to any preceding claim wherein R⁴ is hydrogen, C₁₋₆alkyl or halo.

9. A compound according to any preceding claim wherein R⁵ and R⁶ are independently hydrogen, methyl or Het; or R⁵ and R⁶ together with the nitrogen atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), cyano, -CF₃, hydroxy, -OCF₃, C₁₋₄alkyl and C₁₋₄alkoxy.

10. A compound according to any preceding claim wherein R⁵ and R⁶ are independently hydrogen, methyl or tetrahydropyranyl; or R⁵ and R⁶ together with the nitrogen atom to which they are attached form a morpholine, pyrrolidine, piperazine ring, each of which may be substituted by halo (such as fluoro, chloro, bromo), cyano, -CF₃, hydroxy, -OCF₃, C₁₋₄alkyl or C₁₋₄alkoxy.

11. A compound according to claim 1 wherein
X is N;
R¹ is -NR⁵R⁶, -(CH₂)ₙNR⁵R⁶, -O(CH₂)ₙNR⁵R⁶, -O(CH₂)ₙ-Het, -CONR⁵R⁶, -CO(CH₂)ₙNR⁵R⁶ or -SO₂R⁷;
R² is hydrogen, C₁₋₆alkyl, chloro or fluoro;
R³ is hydrogen or fluoro;
R⁴ is hydrogen, C₁₋₆alkyl or halo;
R⁵ and R⁶ are independently hydrogen, methyl or Het; or R⁵ and R⁶ together with the nitrogen atom to which they are attached form a 3, 4, 5, 6 or 7 membered saturated or unsaturated ring which may contain one or more heteroatoms selected from N, S or O, and wherein the ring may be further substituted by one or more substituents selected from halo (such as fluoro, chloro, bromo), cyano, -CF₃, hydroxy, -OCF₃, C₁₋₄alkyl and C₁₋₄alkoxy;
R⁷ is hydrogen or C₁₋₆alkyl;
Het is a 5 or 6-membered C-linked heterocyclyl group which may be saturated, unsaturated or aromatic, which may contain one or more heteroatoms selected from N, S or O and which may be substituted by C₁₋₆alkyl; and
n is 1-4.

12. A compound according to claim 1 selected from the list:
3-[2-(4-methanesulfonyl-phenyl)-pyridin-4-yl]-2-(6-methyl-pyridin-2-yl)-imidazo[1,2-a]pyridine (Example 6);
3-[2-(4-(morpholin-4-yl)-phenyl)-pyridin-4-yl]-2-pyridin-2-yl-imidazo[1,2-a]pyridine (Example 14);
3-{2-[4-(4-methylpiperazin-1-yl)-phenyl]-pyridin-4-yl}-2-pyridin-2-yl-imidazo[1,2-a]pyridine (Example 15);
2-(6-methyl-pyridin-2-yl)-3-[2-(4-(morpholin-4-ylmethyl)phenyl)-pyridin-4-yl]-imidazo[1,2-a]pyridine (Example 16);
2-(6-methyl-pyridin-2-yl)-3-{2-[4-((morpholin-4-yl)carbonyl)phenyl]-pyridin-4-yl}-imidazo[1,2-*a*]pyridine (Example 29);
2-(pyridin-2-yl)-3-{2-[4-(2-(pyrrolidin-1-yl)ethoxy)phenyl]-pyridin-4-yl}-imidazo[1,2-*a*]pyridine (Example 34);
7-methyl-2-(6-methyl-pyridin-2-yl)-3-{2-[4-(2-(pyrrolidin-1-yl)ethoxy)phenyl]-pyridin-4-yl}-imidazo[1,2-*a*]pyridine (Example 38);
7-methyl-2-(6-methyl-pyridin-2-yl)-3-{2-[4-((1-methyl-imidazol-4-yl)methyloxy)phenyl]-pyridin-4-yl}-imidazo[1,2-*a*]pyridine (Example 41); and
7-methyl-2-(6-methyl-pyridin-2-yl)-3-{2-[4-(aminocarbonylmethyloxy)phenyl]-pyridin-4-yl}-imidazo[1,2-*a*]pyridine (Example 43);
and pharmaceutically acceptable salts, solvates and derivatives thereof.

13. A pharmaceutical composition comprising a compound defined in any preceding claim and a pharmaceutically acceptable carrier or diluent.

14. The use of a compound defined in any one of claims 1 to 12 in the manufacture of a medicament for the treatment or prophylaxis of a disorder mediated by the ALK5 receptor in mammals.

15. The use according to claim 14 wherein the disorder is selected from chronic renal disease, acute renal disease, wound healing, arthritis, osteoporosis, kidney disease, congestive heart failure, ulcers, ocular disorders, corneal wounds, diabetic nephropathy, impaired neurological function, Alzheimer's disease, atherosclerosis, peritoneal and sub-dermal adhesion, any disease wherein fibrosis is a major component, including, but not limited to lung fibrosis, kidney fibrosis, liver fibrosis [for example, hepatitis B virus (HBV), hepatitis C virus (HCV)], alcohol induced hepatitis, retroperitoneal fibrosis, mesenteric fibrosis, haemochromatosis and primary biliary cirrhosis, endometriosis, keloids and restenosis.

16. A compound defined in any one of claims 1 to 12 for use as a medicament.

## Patentansprüche

1. Verbindung der Formel (I), ein pharmazeutisch verträgliches Salz, Solvat oder Derivat davon: 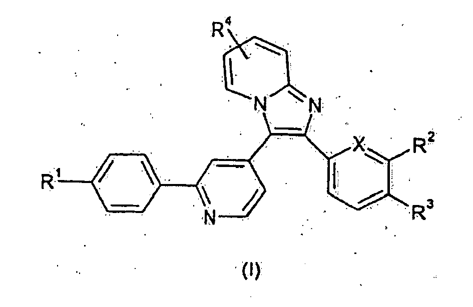 wobei:
X gleich N oder CH ist;
R¹ ausgewählt ist aus Wasserstoff, C₁₋₆-Alkyl, C₁₋₆-Alkenyl, C₁₋₆-Alkoxy, Halogen, Cyano, Perfluor-C₁₋₆-alkyl, Perfluor-C₁₋₆-alkoxy, -NR⁵R⁶, -(CH₂)ₙNR⁵R⁶, -O(CH₂)ₙOR⁷, -O(CH₂)ₙ-Het, -O(CH₂)ₙNR⁵R⁶, -CONR⁵R⁶, -C(O)R⁷, -CO(CH₂)ₙNR⁵R⁶ -SO₂R⁷, -SO₂NR⁵R⁶, -NR⁵SO₂R⁷, -NR⁵COR⁷ und -O(CH₂)ₙCONR⁵R⁶;
R² Wasserstoff, C₁₋₆-Alkyl, Halogen, Cyano oder Perfluor-C₁₋₆-alkyl ist;
R³ Wasserstoff oder Halogen ist;
R⁴ Wasserstoff, Halogen, C₁₋₆-Alkyl oder -NR⁵R⁶ ist;
R⁵ und R⁶ unabhängig ausgewählt sind aus Wasserstoff, C₁₋₆-Alkyl, Perfluor-C₁₋₆-alkyl, Het oder C₁₋₄-Alkoxy-C₁₋₄-alkyl; oder R⁵ und R⁶ zusammen mit dem Stickstoffatom, an das sie gebunden sind, einen 3-, 4-, 5-, 6- oder 7-gliedrigen gesättigten oder ungesättigten Ring bilden, der ein oder mehrere Heteroatome, ausgewählt aus N, S oder 0, enthalten kann und wobei der Ring weiter mit einem oder mehreren Substituenten, ausgewählt aus Halogen (wie z.B. Fluor, Chlor, Brom), Cyano, -CF₃, Hydroxy, -OCF₃, C₁₋₆-Alkyl und C₁₋₆-Alkoxy, substituiert sein kann;
R⁷ Wasserstoff oder C₁₋₆-Alkyl ist;
Het ein 5- oder 6-gliedriger C-gebundener Heterocyclylrest ist, der gesättigt, ungesättigt oder aromatisch sein kann, der ein oder mehrere Heteroatome, ausgewählt aus N, S oder O, enthalten kann und der mit C₁₋₆-Alkyl substituiert sein kann; und
n gleich 1 bis 4 ist.

2. Verbindung nach Anspruch 1, wobei X gleich N ist.

3. Verbindung nach einem vorhergehenden Anspruch, wobei R¹ gleich -NR⁵R⁶, -(CH₂)ₙNR⁵R⁶, -O(CH₂)ₙNR⁵R⁶, -O(CH₂)ₙ-Het, -CONR⁵R⁶, -CO(CH₂)ₙNR⁵R⁶ oder -SO₂R⁷ ist.

4. Verbindung nach einem vorhergehenden Anspruch, wobei R² Wasserstoff, C₁₋₆-Alkyl, Chlor oder Fluor ist.

5. Verbindung nach einem vorhergehenden Anspruch, wobei R³ Wasserstoff oder Fluor ist.

6. Verbindung nach einem vorhergehenden Anspruch, wobei, wenn X gleich N ist, R² Methyl ist.

7. Verbindung nach einem vorhergehenden Anspruch, wobei, wenn X gleich N ist und R² Methyl ist, R³ Wasserstoff ist.

8. Verbindung nach einem vorhergehenden Anspruch, wobei R⁴ Wasserstoff, C₁₋₆-Alkyl oder Halogen ist.

9. Verbindung nach einem vorhergehenden Anspruch, wobei R⁵ und R⁶ unabhängig Wasserstoff, Methyl oder Het sind; oder R⁵ und R⁶ zusammen mit dem Stickstoffatom, an das sie gebunden sind, einen 3-, 4-, 5-, 6- oder 7-gliedrigen gesättigten oder ungesättigten Ring bilden, der ein oder mehrere Heteroatome, ausgewählt aus N, S oder O, enthalten kann und wobei der Ring weiter mit einem oder mehreren Substituenten, ausgewählt aus Halogen (wie z.B. Fluor, Chlor; Brom), Cyano, -CF₃, Hydroxy, -OCF₃, C₁₋₄-Alkyl und C₁₋₄-Alkoxy, substituiert sein kann.

10. Verbindung nach einem vorhergehenden Anspruch, wobei R⁵ und R⁶ unabhängig Wasserstoff, Methyl oder Tetrahydropyranyl sind; oder R⁵ und R⁶ zusammen mit dem Stickstoffatom, an das sie gebunden sind, einen Morpholin-, Pyrrolidin-, Piperazinring bilden, von denen jeder mit Halogen (wie z.B. Fluor, Chlor, Brom), Cyano, -CF₃, Hydroxy, -OCF₃, C₁₋₄-Alkyl oder C₁₋₄-Alkoxy substituiert sein kann.

11. Verbindung nach Anspruch 1, wobei
X gleich N ist;
R¹ gleich -NR⁵R⁶, -(CH₂)ₙNR⁵R⁶, -O(CH₂)ₙNR⁵R⁶, -O(CH₂)ₙ-Het, -CONR⁵R⁶, -CO(CH₂)ₙNR⁵R⁶ oder -SO₂R⁷ ist;
R² Wasserstoff, C₁₋₆-Alkyl, Chlor oder Fluor ist;
R³ Wasserstoff oder Fluor ist;
R⁴ Wasserstoff, C₁₋₆-Alkyl oder Halogen ist;
R⁵ und R⁶ unabhängig Wasserstoff, Methyl oder Het sind; oder R⁵ und R⁶ zusammen mit dem Stickstoffatom, an das sie gebunden sind, einen 3-, 4-, 5-, 6- oder 7-gliedrigen gesättigten oder ungesättigten Ring bilden, der ein oder mehrere Heteroatome, ausgewählt aus N, S oder O, enthalten kann, und wobei der Ring weiter mit einem oder mehreren Substituenten, ausgewählt aus Halogen (wie z.B. Fluor, Chlor, Brom), Cyano, -CF₃, Hydroxy, -OCF₃, C₁₋₄-Alkyl und C₁₋₄-Alkoxy, substituiert sein kann;
R⁷ Wasserstoff oder C₁₋₆-Alkyl ist;
Het ein 5- oder 6-gliedriger C-gebundener Heterocyclylrest ist, der gesättigt, ungesättigt oder aromatisch sein kann, der ein oder mehrere Heteroatome, ausgewählt aus N, S oder
O, enthalten kann und der mit C₁₋₆-Alkyl substituiert sein kann; und
n gleich 1 bis 4 ist.

12. Verbindung nach Anspruch 1, ausgewählt aus der Liste:
3-[2-(4-Methansulfonyl-phenyl)-pyridin-4-yl]-2-(6-methyl-pyridin-2-yl)-imidazo-[1,2-a]pyridin (Beispiel 6);
3-[2-(4-(Morpholin-4-yl)-phenyl)-pyridin-4-yl]-2-pyridin-2-yl-imidazo[1,2-a]pyridin (Beispiel 14);
3- {2-[4-(4-Methylpiperazin-1-yl)-phenyl]-pyridin-4-yl} -2-pyridin-2-yl-imidazo[ 1,2-a]pyridin (Beispiel 15);
2-(6-Methyl-pyridin-2-yl)-3-[2-(4-(morpholin-4-ylmethyl)phenyl)-pyridin-4-yl]-imidazo[1,2-a]pyridin (Beispiel 16);
2-(6-Methyl-pyridin-2-yl)-3-{2-[4-((morpholin-4-yl)carbonyl)phenyl]-pyridin-4-yl } - imidazo[1,2-a]pyridin (Beispiel 29);
2-(Pyridin-2-yl)-3-{2-[4-(2-(pyrrolidin-1-yl)ethoxy)phenyl]-pyridin-4-yl}-imidazo-[1,2-a]pyridin (Beispiel 34);
7-Methyl-2-(6-methyl-pyridin-2-yl)-3- {2-[4-(2-(pyrrolidin-1-yl)ethoxy)phenyl]-pyridin-4-yl}-imidazo[1,2-a]pyridin (Beispiel 38);
7-Methyl-2-(6-methyl-pyridin-2-yl)-3- {2-[4-((1-methyl-imidazol-4-yl)methyloxy)phenyl]-pyridin-4-yl}-imidazo[1,2-a]pyridin (Beispiel 41); und
7-Methyl-2-(6-methyl-pyridin-2-yl)-3-{2-[4-(aminocarbonylmethyloxy)phenyl]-pyridin-4-yl}-imidazo[1,2-a]pyridin (Beispiel 43);
und pharmazeutisch verträgliche Salze, Solvate und Derivate davon.

13. Arzneimittel, umfassend eine in einem vorhergehenden Anspruch definierte Verbindung und einen pharmazeutisch verträglichen Träger oder ein pharmazeutisch verträgliches Verdünnungsmittel.

14. Verwendung einer in einem der Ansprüche 1 bis 12 definierten Verbindung bei der Herstellung eines Medikaments zur Behandlung oder Prophylaxe einer durch den ALK5-Rezeptor vermittelten Störung bei Säugern.

15. Verwendung nach Anspruch 14, wobei die Störung ausgewählt ist aus chronischer Nierenerkrankung, akuter Nierenerkrankung, Wundheilung, Arthritis, Osteoporose, Nierenerkrankung, dekompensierter Herzinsuffizienz, Geschwüren, Augenerkrankungen, Hornhautverletzungen, diabetischer Nephropathie, eingeschränkter neurologischer Funktion, Alzheimer-Krankheit, Atherosklerose, peritonealer und subkutaner Adhäsion, jeglicher Erkrankung, bei der Fibrose eine Hauptkomponente darstellt, einschließlich, jedoch nicht beschränkt auf Lungenfibrose, Nierenfibrose, Leberfibrose [z.B. Hepatitis B-Virus (HBV), Hepatitis C-Virus (HCV)], Alkohol-induzierter Hepatitis, retroperitonealer Fibrose, Mesenterialfibrose, Hämochromatose und primärer Gallenzirrhose, Endometriose, Wulstnarben und Restenose.

16. In einem der Ansprüche 1 bis 12 definierte Verbindung zur Verwendung als Medikament.

## Revendications

1. Composé de formule (I), ou un de ses sels, produits de solvatation ou dérivés pharmaceutiquement acceptables : 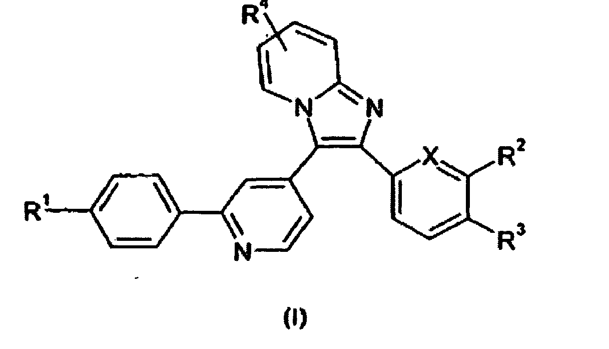 formule dans laquelle
X représente N ou un groupe CH ;
R¹ est choisi entre un atome d'hydrogène ou des groupes alkyle en C₁ à C₆, alcényle en C₁ à C₆, alkoxy en C₁ à C₆, halogéno, cyano, perfluoralkyle en C₁ à C₆, perfluoralkoxy en C₁ à C₆, -NR⁵R⁶, (CH₂)ₙNR⁵R⁶, -O(CH₂)ₙOR⁷, -O(CH₂)ₙ-Het, -O(CH₂)ₙNR⁵R⁶, -CONR⁵R⁶, -C(O)R⁷, -CO(CH₂)ₙR⁵R⁶, -SO₂R⁷, -SO₂NR⁵R⁶, -NR⁵SO₂R⁷, -NR⁵COR⁷ et -O(CH₂)ₙCONR⁵R⁶ ;
R² représente un atome d'hydrogène, un groupe alkyle en C₁ à C₆, halogéno, cyano ou perfluoralkyle en C₁ à C₆ ;
R³ représente un atome d'hydrogène ou un groupe halogéno ;
R⁴ représente un atome d'hydrogène, un groupe halogéno, alkyle en C₁ à C₆ ou -NR⁵R⁶ ;
R⁵ et R⁶ sont choisis indépendamment entre un atome d'hydrogène, des groupes alkyle en C₁ à C₆, perfluoralkyle en C₁ à C₆, Het et (alkoxy en C₁ à C₄) (alkyle en C₁ à C₄) ; ou bien R⁵ et R⁶, conjointement avec l'atome d'azote auquel ils sont fixés, forment un noyau tri-, tétra-, penta-, hexa- ou heptagonal saturé ou insaturé qui peut contenir un ou plusieurs hétéroatomes choisis entre N, S et O, le noyau pouvant être en outre substitué avec un ou plusieurs substituants choisis entre des substituants halogéno (comme fluoro, chloro, bromo), cyano, -CF₃, hydroxy, -OCF₃, alkyle en C₁ à C₆ et alkoxy en C₁ à C₆ ;
R⁷ représente un atome d'hydrogène ou un groupe alkyle en C₁ à C₆ ;
Het représente un groupe hétérocyclyle penta- ou hexagonal lié à C, qui peut être saturé, insaturé ou aromatique, qui peut contenir un ou plusieurs hétéroatomes choisis entre N, S et O et qui peut être substitué avec un substituant alkyle en C₁ à C₆ ; et
n a une valeur de 1 à 4.

2. Composé suivant la revendication 1, dans lequel X représente N.

3. Composé suivant l'une quelconque des revendications précédentes, dans lequel R¹ représente un groupe -NR⁵R⁶, -(CH₂)ₙNR⁵R⁶, -O(CH₂)ₙRN⁵R⁶, -O(CH₂)ₙ-Het, CONR⁵R⁶, -CO(CH₂)ₙNR⁵R⁶ ou -SO₂R⁷.

4. Composé suivant l'une quelconque des revendications précédentes, dans lequel R² représente un atome d'hydrogène, un groupe alkyle en C₁ à C₆, chloro ou fluoro.

5. Composé suivant l'une quelconque des revendications précédentes, dans lequel R³ représente un atome d'hydrogène ou un groupe fluoro.

6. Composé suivant l'une quelconque des revendications précédentes, dans lequel, lorsque X représente N, R² représente un groupe méthyle.

7. Composé suivant l'une quelconque des revendications précédentes, dans lequel, lorsque X représente N et R² représente un groupe méthyle, R³ représente un atome d'hydrogène.

8. Composé suivant l'une quelconque des revendications précédentes, dans lequel R⁴ représente un atome d'hydrogène, un groupe alkyle en C₁ à C₆ ou halogéno.

9. Composé suivant l'une quelconque des revendications précédentes, dans lequel R⁵ et R⁶ représentent indépendamment un atome d'hydrogène, un groupe méthyle ou Het ; ou bien R⁵ et R⁶, conjointement avec l'atome d'azote auquel ils sont fixés, forment un noyau tri-, tétra-, penta-, hexa- ou heptagonal saturé ou insaturé qui peut contenir un ou plusieurs hétéroatomes choisis entre N, S et O, le noyau pouvant être en outre substitué avec un ou plusieurs substituants choisis entre des substituants halogéno, (comme fluoro, chloro, bromo), cyano, -CF₃, hydroxy, -OCF₃, alkyle en C₁ à C₄ et alkoxy en C₁ à C₄.

10. Composé suivant l'une quelconque des revendications précédentes, dans lequel R⁵ et R⁶ représentent indépendamment un atome d'hydrogène, un groupe méthyle ou tétrahydropyrannyle ; ou bien R⁵ et R⁶, conjointement avec l'atome d'azote auquel ils sont fixés, forment un noyau morpholine, pyrrolidine ou pipérazine, chacun pouvant être substitué avec des substituants halogéno (comme fluoro, chloro, bromo), cyano, -CF₃, hydroxy, -OCF₃, alkyle en C₁ à C₄ ou alkoxy en C₁ à C₄.

11. Composé suivant la revendication 1, dans lequel
X représente N ;
R¹ représente un groupe -NR⁵R⁶, -(CH₂)ₙNR⁵R⁶, -O(CH₂)ₙR⁵R⁶, -O(CH₂)ₙ₋Het, -CONR⁵R⁶, -CO(CH₂)ₙTR⁵R⁶ ou -SO₂R⁷ ;
R² représente un atome d'hydrogène ou un groupe alkyle en C₁ à C₆, chloro ou fluoro ;
R³ représente un atome d'hydrogène ou un groupe fluoro ;
R⁴ représente un atome d'hydrogène, un groupe alkyle en C₁ à C₆ ou halogéno ;
R⁵ et R⁶ représentent indépendamment un atome d'hydrogène, un groupe méthyle ou Het ; ou bien R⁵ et R⁶, conjointement avec l'atome d'azote auquel ils sont fixés, forment un noyau tri-, tétra-, penta-, hexa- ou heptagonal saturé ou insaturé qui peut contenir un ou plusieurs hétéroatomes choisis entre N, S et O, le noyau pouvant être en outre substitué avec un ou plusieurs substituants choisis entre des substituants halogéno (comme fluoro, chloro, bromo), cyano, -CF₃, hydroxy, -OCF₃, alkyle en C₁ à C₄ et alkoxy en C₁ à C₄ ;
R⁷ représente un atome d'hydrogène ou un groupe alkyle en C₁ à C₆ ;
Het représente un groupe hétérocyclyle penta- ou hexagonal lié à C, qui peut être saturé, insaturé ou aromatique, qui peut contenir un ou plusieurs hétéroatomes choisis entre N, S et O et qui peut être substitué avec un substituant alkyle en C₁ à C₆ ; et
n a une valeur de 1 à 4.

12. Composé suivant la revendication 1, choisi dans la liste :
3-[2-(4-méthanesulfonyl-phényl)-pyridine-4-yl]-2-(6-méthyl-pyridine-2-yl)-imidazo[1,2-a]pyridine (exemple 6) ;
3-[2-(4-morpholin-4-yl)-phényl)-pyridine-4-yl]-2-pyridine-2-yl-imidazo[1,2-a]pyridine (exemple 14) ;
3-{2-[4-(4-méthylpipérazine-1-yl)-phényl]-pyridine-4-yl}-2-pyridine-2-yl-imidazo[1,2-a]pyridine (exemple 15) ;
2-(6-méthyl-pyridine-2-yl)-3-[2-(4-(morpholin-4-ylméthyl)phényl)-pyridine-4-yl]-imidazo[1,2-a]pyridine (exemple 16) ;
2-(6-méthyl-pyridine-2-yl)-3-{2- [4- ((morpholin-4-yl)carbonyl)phényl]-pyridine-4-yl}-imidazo[1,2-*a*]pyridine (exemple 29) ;
2-(pyridine-2-yl)-3-{2-[4-(2-(pyrrolidine-1-yl)-éthoxy)phényl]-pyridine-4-yl}-imidazo[1,2-*a*]pyridine (exemple 34) ;
7-méthyl-2-(6-méthyl-pyridine-2-yl)-3-{2-[4-(2-(pyrrolidine-1-yl)éthoxy)phényl]-pyridine-4-yl}-imidazo[1,2-a] pyridine (exemple 38) ;
7-méthyl-2-(6-méthyl-pyridine-2-yl) -3-{2-[4- ((1-méthyl-imidazol-4-yl) méthyloxy) phényl]-pyridine-4-yl}-imidazo[1, 2-a]pyridine (exemple 41) ; et
7-méthyl-2- (6-méthyl-pyridine-2-yl) -3-{2- [4 (aminocarbonyl-méthyloxy)phényl]-pyridine-4-yl}-imidazo[1,2-a]pyridine (exemple 43) ;
et leurs sels, produits de solvatation et dérivés pharmaceutiquement acceptables.

13. Composition pharmaceutique comprenant un composé défini dans l'une quelconque des revendications précédentes et un support ou diluant pharmaceutiquement acceptable.

14. Utilisation d'un composé défini dans l'une quelconque des revendications 1 à 12 dans la production d'un médicament destiné au traitement ou à la prophylaxie d'une affection à médiation par le récepteur ALK5 chez des mammifères.

15. Utilisation suivant la revendication 14, dans laquelle l'affection est choisie entre une maladie rénale chronique, une maladie rénale aiguë, la cicatrisation d'une plaie, l'arthrite, l'ostéoporose, une maladie rénale, l'insuffisance cardiaque congestive, les ulcères, des troubles oculaires, des plaies de la cornée, la néphropathie diabétique, une altération de la fonction neurologique, la maladie d'Alzheimer, l'athérosclérose, une adhérence péritonéale et une adhérence sous-dermique, n'importe quelle maladie dont la fibrose est une composante essentielle, comprenant, mais à titre non limitatif, la fibrose pulmonaire, la fibrose rénale, la fibrose hépatique, [provoquée par le virus de l'hépatite B (HBV) ou le virus de l'hépatite C (HCV)], l'hépatite induite par l'alcool, la fibrose rétropéritonéale, la fibrose mésentérique, l'hémochromatose et la cirrhose biliaire primaire, l'endométriose, la formation de chéloïdes et une resténose.

16. Composé suivant l'une quelconque des revendications 1 à 12, destiné à être utilisé comme médicament.
